# EUROPÄISCHE PATENTSCHRIFT

(11) **EP 3 417 897 B1**
(45) Veröffentlichungstag und Bekanntmachung des Hinweises auf die Patenterteilung: **09.09.2020**
(21) Anmeldenummer: 18000526.6
(22) Anmeldetag: 15.06.2018
(51) Int. Cl.: A61M 16/06, A61M 16/08

(54) **ATEMMASKE MIT EINER ATEMGASÖFFNUNG**
RESPIRATORY MASK WITH A BREATHING GAS OPENING
MASQUE RESPIRATOIRE DOTÉ D'UNE OUVERTURE DE GAZ RESPIRATOIRE

(30) Priorität: 19.06.2017 DE 102017005692; 19.06.2017 DE 102017005704
(43) Veröffentlichungstag der Anmeldung: 26.12.2018
(73) Patentinhaber: Löwenstein Medical Technology S.A., 2557 Luxembourg (LU)
(72) Erfinder: Frerichs, Arnold, 21614 Buxtehude (DE); Gardein, Joachim, E-38430 Icod de Ios Vinos, Tenerife/Islas Canarias (ES)
(74) Vertreter: Marx, Thomas

(56) Entgegenhaltungen:
- WO-A1-2013/006899
- DE-A1-102005 031 541
- US-A1- 2008 072 910

## Beschreibung

Die Erfindung betrifft eine Atemmaske mit einem Maskenkörper und einem gelenkigen Anschlussstück, welches mit einem Atemschlauch verbindbar ist.

Atemmasken werden beispielsweise im Zusammenhang mit Beatmungsgeräten verwendet, um Atemgas zum Patienten zu leiten und eine Ableitung des ausgeatmeten Atemgases zu unterstützen. Die Atemmaske ist typischerweise über einen Atemschlauch mit einem Beatmungsgerät verbunden.

Ein Nachteil bekannter Atemmasken war, die beim Ausatmen durch den Maskenkörper und den Beatmungsschlauch entstehende, für den Patienten und die Umgebung unangenehme, akustische Beeinträchtigung. Zudem verursachte der ausgeatmete Luftstrom einen am Patienten entlang streichenden kühlen Luftzug. Diese Herausforderungen wurden durch eine Adaption der Maske gemäß DE 10 2005 041 717 A1 und EP 1 632 262 B1 mittels geeigneter Anordnung und Struktur von Ausatemspalten zwischen Maskenkörper und Sicherungsring gemeistert.
Bei der Maske der EP 1 632 262 B1 ist die Stirnstütze fest und unlösbar mit dem Maskenkörper verbunden. Ein separater Sicherungsring hält das Kugelgelenk im Maskenkörper. Zwischen dem Sicherungsring und dem Maskenkörper werden Ausatemspalte bereitgestellt.

Bei der Maske der DE 10 2005 041 717 A1 befindet sich das Bajonett am Sicherungsring und rastet in Fügerichtung in den Maskenkörper ein.

Erfindungsgemäß befindet sich das Bajonett an der Stirnstütze und rastet radial nach außen, in korrespondierende Aufnahme am inneren Radius im Maskenkörper ein. Die radiale Verrastung, erleichtert die Findung und Führung. Der Ausatemspalt zwischen Stirnstütze und Maskenkörper ist zudem unbeeinflusst von Maßabweichung des Bajonetts.

Fertigungs- und wartungstechnischer Nachteil ist, dass die Luft in den bekannten Atemmasken durch Durchbrüche im Maskenkörper hindurch zu den Ausatemspalten geleitet wird. Zudem schwächen Durchbrüche die mechanische Stabilität des Maskenkörpers.

Des Weiteren bestehen die Masken aus vielen Einzelteilen, die zwar eine erhöhte Anpassbarkeit an den Patienten erlauben, jedoch komplex zu montieren und teuer in der Fertigung sind. Der zusätzliche Sicherungsring benötigt Bauraum.

Bekannte Masken stellen den Raum zur Aufnahme der Atemöffnungen des Patienten überwiegend innerhalb des Maskenkörpers zur Verfügung. Dies sorgt für einen erhöhten Materialbedarf im Bereich des Maskenkörpers, da dieser ein größeres Volumen umschließen muss.

Weiterhin stellt bei bekannten Atemmasken die Ankopplung der Bänderung zwecks sicherer Fixierung der Maske am Kopf des Patienten während des Aufsetzens der Maske häufig hohe Anforderungen an die Fingerfertigkeit des Patienten, da die Sicht auf die entsprechenden Verbindungsteile nicht gegeben ist.

Die WO2013006899 A1 offenbart eine Atemmaske mit einem Maskenkörper und einer Öffnung für Atemgase, und einem Verbinder mit einer äußeren Kontur der zumindest mit der äußeren Kontur teilweise in die Öffnung eingeführt ist und mechanisch und lösbar in der Öffnung gehalten wird, wobei ein Anschlußstück für die Zuleitung von Atemgas lösbar mit dem Verbinder verbunden ist, wobei Ausströmflächen für Ausatemgase in der Schnittstelle zwischen der innenliegenden Oberfläche der Öffnung und der äußeren Kontur des Verbinders und Distanzelementen zwischen der innenliegenden Oberfläche der Öffnung und der äußeren Kontur des Verbinders, die die Höhe von Dimensionierung der Ausströmflächen vorgeben.

Die Masken der US2008072910 A1 und D3 DE102005031541 A1 weisen zusätzlich zu den Merkmalen der WO2013006899 A1 jeweils noch Stirnstützen auf, die separate Aufnahmen oder Befestigungspunkte am Maskenkörper haben.

Eine erste Aufgabe der Erfindung ist es, die Leitstrukturen für die ausgeatmeten Atemgase zu den Ausatemspalten so anzupassen, dass keine Durchbrüche im Maskenkörper erforderlich sind.

Diese Aufgabe wird erfindungsgemäß dadurch gelöst, dass die Leitstrukturen durch Aussparungen im Raum zwischen Maskenkörper und einem Stirnstützenverbinder gebildet werden.
Bei dieser Erfindung werden lediglich zwei Teile verwendet; eine nicht verstellbare Stirnstütze und ein Maskenkörper. Dadurch wird ein schlanker, materialsparender Aufbau erreicht.
Bei dieser Erfindung sind ein Teil des Kugelkäfigs und der Sicherungsring einteilig mit der Stirnstütze ausgeführt. Die Stirnstütze hält und führt das Kugelgelenk.

Die wesentliche Aufgabe der Erfindung ist es, eine Maske mit Stirnstütze bereitzustellen, die einen schlanken, materialsparenden Aufbau ermöglicht und die Anzahl der benötigten Einzelteile zu reduzieren, um so eine kostengünstige Fertigung und eine einfache Montage der Atemmaske zu ermöglichen.

Erfindungsgemäß wird die Aufgabe gelöst durch eine Atemmaske mit einem Maskenkörper und einer Öffnung für Atemgase, und einem Verbinder mit einer äußeren Kontur der zumindest mit der äußeren Kontur teilweise in die Öffnung eingeführt ist und mechanisch und lösbar in der Öffnung gehalten wird, wobei ein Anschlußstück für die Zuleitung von Atemgas lösbar mit dem Verbinder verbunden ist, wobei Ausströmflächen für Ausatemgase in der Schnittstelle zwischen der innenliegenden Oberfläche der Öffnung und der äußeren Kontur des Verbinders und Distanzelementen zwischen der innenliegenden Oberfläche der Öffnung und der äußeren Kontur des Verbinders, die die Höhe von Dimensionierung der Ausströmflächen vorgeben, wobei der Verbinder ein integraler Teil der Stirnstütze ist und deren Maskenkörper eine zentrale, runde Öffnung aufweist, die der Aufnahme der Stirnstütze dient und so eine Schnittstelle zu der Stirnstütze bildet.

Die Atemmaske ist auch dadurch gekennzeichnet, dass der Verbinder ein integraler Teil der Stirnstütze ist.

Die Atemmaske ist auch dadurch gekennzeichnet, dass der Maskenkörper rotationssymmetrisch ausgeführt ist.

Die Atemmaske ist auch dadurch gekennzeichnet, dass die Öffnung rund ist und einen inneren Durchmesser von zumindest 1 cm aufweist.

Die Atemmaske ist auch dadurch gekennzeichnet, dass die Öffnung eine Länge von zumindest von 4 mm aufweist.

Die Atemmaske ist auch dadurch gekennzeichnet, dass die Öffnung einen Umfang zumindest von 2 cm aufweist.

Die Atemmaske ist auch dadurch gekennzeichnet, dass der Maskenkörper eine zentrale, runde Öffnung aufweist, die der Aufnahme der Stirnstütze dient und so eine Schnittstelle zu der Stirnstütze bildet.

Die Atemmaske ist auch dadurch gekennzeichnet, dass die Öffnung des Maskenkörpers in einem Bereich durch ein Rohr konstanten Durchmessers definiert wird.

Die Atemmaske ist auch dadurch gekennzeichnet, dass der rohrförmiger Bereich mehr als 4 mm lang ist.

Die Atemmaske ist auch dadurch gekennzeichnet, dass der rohrförmiger Bereich in einem Winkel von 20 - 90° relativ zum rohrförmigen Bereich nach außen abknickt und einen trichterförmigen Bereich bildet.

Die Atemmaske ist auch dadurch gekennzeichnet, dass sie mehrere Ausströmflächen auf der innenliegenden Oberfläche der Öffnung aufweist, die von Distanzelementen, wie Rippen, unterbrochen sind.

Die Atemmaske ist auch dadurch gekennzeichnet, dass die Distanzelemente sich entlang innenliegende Oberfläche des Maskenkörpers von innen nach außen erstrecken und so die Atemgase vom Patienten weg nach Außen leiten oder die Distanzelemente sich auch entlang des trichterförmigen Bereiches erstrecken.

Die Atemmaske ist auch dadurch gekennzeichnet, dass die Stirnstütze als Einheit mit einer Gelenkaufnahme für ein Anschlussstück ausgeführt ist.

Die Atemmaske ist auch dadurch gekennzeichnet, dass die Ausströmstruktur für ausgeatmete Gase durch die Oberflächen der Öffnung des Maskenkörpers, und der Stirnstütze definiert ist.

Die Atemmaske ist auch dadurch gekennzeichnet, dass die Ausatemspalte gleichzeitig als Aufnahme für eine mechanische Verbindung von Maskenkörper und Sicherungsring oder Stirnstützenmodul dienen.

Die Atemmaske ist auch dadurch gekennzeichnet, dass im Umfang der Öffnung mindestens zwei Kanäle angeordnet sind, die als Aussparung im rohrförmigen Bereich und/oder im trichterförmigen Bereich realisiert sind oder die Kanäle als Aufnahmen für die mechanische Ankopplung der Stirnstütze fungieren, die beispielsweise als Bajonettverschluss realisiert werden kann.

Die Atemmaske ist auch dadurch gekennzeichnet, dass die Kanäle in doppelter Funktion sowohl als Aufnahmen für die mechanische Ankopplung der Stirnstütze, als auch als Abströmkanäle für ausgeatmete Atemgase dienen.

Die Atemmaske ist auch dadurch gekennzeichnet, dass die Kanäle als Aufnahmen für die mechanische Ankopplung der Stirnstütze fungieren, die als Bajonettverschluss realisiert ist und bei der für den Bajonettverschluss im hinteren Bereich neben jedem Kanal jeweils ein Zahn vorgesehen ist, der radial nach innen aus der inneren Kontur der Öffnung herausragt und der den benötigten mechanischen Widerstand für den Verschluss bietet.

Die Atemmaske ist auch dadurch gekennzeichnet, dass die mechanische Verrastung zwischen Maskenkörper und Stirnstütze in radialer Richtung durch radial zur inneren Kontur der Öffnung angeordnete Rastelemente erfolgt.

Die Atemmaske ist auch dadurch gekennzeichnet, dass auf der inneren Oberfläche der Öffnung mehrere Distanzrippen angeordnet sind, die eine Verbindung von Maskenkörper und Stirnstütze ohne Spiel bzw. unter Vorspannung ermöglichen.

Die Atemmaske ist auch dadurch gekennzeichnet, dass die Distanzrippen im Bereich 100 - 500 *µ*m sind und so bemessen sind, dass sie einen Spalt für die Atemgase bilden.

Die Atemmaske ist auch dadurch gekennzeichnet, dass die Distanzrippen unterschiedliche Höhen aufweisen oder einen graduellen Anstieg der Höhe.

Die Atemmaske ist auch dadurch gekennzeichnet, dass die Distanzrippen innen, im rohrförmigen Bereich weniger hoch sind, beispielsweise 100 - 299 *µ*m, und im trichterförmigen Bereich höher, beispielsweise 300 - 500 *µ*m.

Die Atemmaske ist auch dadurch gekennzeichnet, dass im Bereich der Abströmkanäle das Material im Ring- und Trichterbereich entsprechend der Höhe der Distanzrippen verstärkt ist.

Die Atemmaske ist auch dadurch gekennzeichnet, dass die Distanzrippen und die verstärkten Bereiche seitlich die Ausströmflächen begrenzen.

Die Atemmaske ist auch dadurch gekennzeichnet, dass die verstärken Bereich oben und/oder unten angeordnet sind, also in den Bereichen die in die Augenregion bzw. zum Mund des Anwenders weisen.

Die Atemmaske ist auch dadurch gekennzeichnet, dass im Maskenkörper sichtbare und/oder fühlbare Strukturen ausgebildet sein, die radial von den Ausströmkanälen wegführen und so die Abströmrichtung der Atemgase anzeigen.

Die erfindungsgemäße Atemmaske kann als beliebiges gasführendes Patienteninterface ausgeführt sein.

Diese Aufgabe wird erfindungsgemäß dadurch gelöst, dass die erfindungsgemäße Stirnstütze mehrere Funktionen vereint. So sind in einem einzigen Teil die Funktionen der Stirnstütze, des Sicherungsringes und des Kugelgelenks zur Aufnahme eines Anschlussstücks kombiniert. Zusätzlich definiert der Stirnstützenverbinder durch seine Kontur gemeinsam mit dem Maskenkörper eine Leitstruktur für die ausgeatmeten Atemgase eines Patienten.

Die Montage der Atemmaske kann zusätzlich dadurch vereinfacht werden, dass der Maskenkörper rotationssymmetrisch ausgeführt wird, wodurch dieser in zwei zueinander um 180° gedrehten Positionen funktionsgleich verbaut werden kann.

Eine weitere Aufgabe der Erfindung ist es Material und Fertigungsaufwand am Maskenkörper einzusparen.

Diese Aufgabe wird dadurch erfüllt, dass der an den Atemöffnungen des Patienten benötigte Raum innerhalb des Maskenwulstes zur Verfügung gestellt wird.
Weiter ist es eine Aufgabe der Erfindung die Bandankopplung an die Atemmaske zur erleichtern.

Diese Aufgabe wird erfindungsgemäß durch eine Anpassung des mechanischen Aufbaus der Bandankopplung in der Form erfüllt, dass eine Führungsstruktur im Maskenkörper integriert wird, die die auf der Bandseite vorhandene Kopplungsstruktur im Ankopplungsvorgang helfend zum Fixierpunkt führt. Dadurch wird die Ankopplung ohne Sicht wesentlich vereinfacht.

Des Weiteren wird diese Aufgabe auch dadurch erfüllt, dass die Stirnstütze verschiedene Optionen für die Bandankopplung bietet.

Die erfindungsgemäße Atemmaske, die nicht nur als Einzelteil, sondern auch als Element eines ganzen Beatmungsgerätes zu verstehen ist, ist bevorzugt modular aufgebaut. Diese Modularität umfasst vier wesentliche Bestandteile, die durch einen Maskenkörper, eine Stirnstütze, einen Maskenwulst und ein gelenkiges Anschlussstück gegeben sind. Das Anschlussstück ist mit einem Atemschlauch verbindbar, der das Atemgas zwischen Atemmaske und Beatmungsgerät transportiert. Weiterhin zeichnet sich die erfindungsgemäße Atemmaske durch die Integration von mindestens einem Ausatemspalt aus, der von bestimmten Bereichen der Oberflächen von Maskenkörper und Stirnstütze zwischen diesen begrenzt wird. Zur Fixierung der Atemmaske am Kopf eines Patienten sind Bänder vorgesehen, für die die erfindungsgemäße Atemmaske Ankopplungsstrukturen zur Verfügung stellt.

Der Maskenkörper dient als Basis der Atemmaske und stellt Verbindungsstrukturen zur Stirnstütze, zum Maskenwulst und zu der Bänderung zur Verfügung. Die Verbindungsstrukturen zu Maskenwulst und Stirnstütze sind bevorzugt mechanisch kodiert, um eine definierte Positionierung der Elemente zueinander sicherzustellen. Als Verbindungsform von Maskenkörper und Stirnstütze ist insbesondere an einen Bajonettverschluss mit Einrastfunktion gedacht. Die Verbindung von Maskenkörper und Maskenwulst kann beispielsweise durch eine Steckverbindung mit Hinterschnitt oder Zweikomponentenverklebung realisiert werden.

Eine vorteilhafte Ausführungsform des Maskenkörpers ist es, diesen rotationssymmetrisch auszulegen. So kann der Maskenkörper funktional identisch in zwei zueinander um 180° gedrehten Positionen in der Atemmaske verbaut werden. Jedoch sind auch, zum Beispiel zwecks mechanischer Kodierung oder der Gestaltung von Ausatemspalten, nicht vollständig rotationssymmetrische Maskenkörper für eine erfindungsgemäße Atemmaske denkbar.

An seinen Seiten weist der Maskenkörper Ankopplungsstrukturen für Bänder auf. Insbesondere ist dabei an die Kombination einer Führungsstruktur, die das Gegenstück zur Ankopplung auf Bandseite zum Fixierpunkt führt, mit einer Fixiermechanik gedacht. Die Fixiermechanik kann erfindungsgemäß beispielsweise als Federplatte mit einem Zapfen ausgeführt sein, während eine trichterförmige Führungsstruktur das bänderseitige Koppelstück in den durch Federplatte und Zapfen definierten Rastpunkt führt.

Die Grundform der inneren Oberfläche des Maskenkörpers ist zum Beispiel ringförmig ausgelegt und öffnet sich auf der einem Patienten abgewandten Seite trichterförmig. Weiterhin weist die Oberfläche im inneren Radius des Maskenkörpers Strukturen auf, die der mechanischen Sicherung der Verbindung mit der Stirnstütze und der Begrenzung von einer oder mehreren Atemgas-Leitstrukturen sowie mindestens einem Ausatemspalt dienen. Diese Strukturen können beispielsweise durch Distanzrippen gegeben sein, die eine Verbindung von Maskenkörper und Stirnstütze ohne Spiel erlauben und die Ausatemspalte begrenzen. Weiterhin sind Strukturen vorgesehen, die eine sichere Verbindung und Fixierung von Maskenkörper und Stirnstütze ermöglichen. Insbesondere ist dabei an kanalartige Aussparungen in zur radialen Ebene orthogonaler Richtung gedacht, die als Führung für Bajonettzähne am Verbindungsstück der Stirnstütze dienen. Des Weiteren dienen die nach dem Verschrauben von Maskenkörper und Stirnstütze freiwerdenden Kanäle in einer vorteilhaften Ausführungsform der erfindungsgemäßen Atemmaske als Leitung für die ausgeatmeten Atemgase eines Patienten vom Inneren der Atemmaske zum Trichter.

Die trichterförmige Öffnung erlaubt es die ausgeatmeten Atemgase eines Patienten in einem vorteilhaften Winkel durch mindestens einen Ausatemspalt an der vom Patienten abgewandten Seite aus der Atemmaske abzuleiten.

Vorteilhaft ist eine Formgebung innerhalb der trichterförmigen Öffnung des Maskenkörpers, die Ausatemspalte in Verbindung mit der montierten Stirnstütze in definierten Bereichen automatisch verschließt. Zum Beispiel ist dabei an den Bereich gedacht, in dem Ausatemgase direkt oder abgelenkt durch Teile der Atemmaske in Richtung der Augen austreten können, da dies von Patienten als besonders unangenehm wahrgenommen wird.

Jedoch ist auch an Ausführungsformen gedacht, die im nach oben gerichteten Bereich des Trichters mindestens einen Ausatemspalt aufweisen. Auch können Ausführungsformen der erfindungsgemäßen Atemmaske zusätzlich die weitere Struktur der Stirnstütze insbesondere im Bereich des Stegs als führende Oberfläche für das entweichende Atemgas nutzen.

Der Maskenwulst dient der Aufnahme der Atemöffnungen und des Abdichtens der Maske am Gesicht eines Patienten. Der Maskenwulst kann aus einem relativ weichen Material gefertigt sein, das zum Zwecke der Abdichtung am Gesicht eines Patienten und zur Montage an dem Maskenkörper eine bestimmte Elastizität aufweisen kann. Besonders ist an Kunststoff als Material des Maskenwulstes gedacht. Die Grundform des Maskenwulstes ist bevorzugter Weise an die menschliche Anatomie angepasst und für Nasalmasken entsprechend triangulär vorgesehen. An der vom Patienten abgewandten Seite befindet sich eine Verbindungsöffnung, die in Größe und Form an die Verbindungsform des Maskenkörpers angepasst ist. Durch die Kombination von verschiedenen Formen bei weiterhin angepassten Umfängen auf Seiten von Maskenwulst und Maskenkörper, lassen sich im relativ weichen Material des Maskenwulstes die Steifigkeit und das Verhältnis von Breite und Höhe einstellen. Denkbar sind zum Beispiel eine ovale Ausführung der Wulstaufnahme am Maskenkörper und eine kreisrunde Ausführung der Öffnung am Maskenwulst.

Die Stirnstütze der erfindungsgemäßen Atemmaske ist einteilig ausgeführt und vereint weitere Funktionen. Diese Funktionen sind die Vervollständigung der Leitstruktur für die ausgeatmeten Atemgase eines Patienten in Kombination mit den im Maskenkörper angelegten Merkmalen, insbesondere der trichterförmigen Zuleitung der Atemgase zu dem mindestens einen Ausatemspalt, die gelenkige Anbindung des Anschlussstückes, beispielsweise durch einen integrierten Kugelkäfig, sowie eine Bandankopplung.

Die Erfindung wird im Folgenden anhand der Figuren in beispielhafter Ausführungsform erläutert. Es zeigen:
Fig. 1: eine als Nasalmaske ausgeführte Atemmaske in seitlicher Ansicht,
Fig. 2 : die in Fig. 1 gezeigte Atemmaske von vorn,
Fig. 3 : die in Fig. 1 gezeigte Atemmaske von hinten,
Fig. 4 : die in Fig. 1 gezeigte Atemmaske von oben,
Fig. 5: die in Fig. 1 gezeigte Atemmaske von unten,
Fig. 6: einen vertikalen Schnitt der in Fig. 1 gezeigten Atemmaske,
Fig. 7: eine perspektivische Darstellung einer erfindungsgemäßen Ausführungsform eines Maskenkörpers von vorn,
Fig. 8: eine perspektivische Darstellung des in Fig. 7 gezeigten Maskenkörpers von hinten,
Fig. 9: eine seitliche Ansicht des Maskenkörpers aus Fig. 7,
Fig. 10: eine Ansicht des Maskenkörpers aus Fig. 7 von oben,
Fig. 11: eine Ansicht des Maskenkörpers aus Fig. 7 von vorn,
Fig. 12: eine Ansicht des Maskenkörpers aus Fig. 7 von hinten,
Fig. 13: einen vertikalen Schnitt durch den Maskenkörper aus Fig. 7,
Fig. 14: einen horizontalen Schnitt durch den Maskenkörper aus Fig. 7,
Fig. 15: eine perspektivische Ansicht einer erfindungsgemäßen Ausführungsform der Stirnstütze von vorn,
Fig. 16: eine perspektivische Ansicht einer Stirnstütze aus Fig. 15 von hinten,
Fig. 17: eine Ansicht der Stirnstütze aus Fig. 15 von unten,
Fig. 18: eine Ansicht der Stirnstütze aus Fig. 15 von oben,
Fig. 19: eine perspektivische Ansicht eines Moduls einer erfindungsgemäßen Beatmungsvorrichtung bestehend aus Maskenkörper und Stirnstütze,
Fig. 20: eine Seitenansicht des in Fig. 19 gezeigten Moduls,
Fig. 21: eine perspektivische Ansicht eines erfindungsgemäßen Anschlussstücks,
Fig. 22: eine seitliche Ansicht eines erfindungsgemäßen Anschlussstücks,
Fig. 23: einen vertikalen Schnitt des Anschlussstücks aus Fig. 21,
Fig. 24: eine perspektivische Darstellung eines erfindungsgemäßen Clips für die Bandaufnahme und die Verbindung mit dem Maskenkörper von der der Maske abgewandten Seite,
Fig. 25: eine perspektivische Darstellung des Clips aus Fig. 24 von der der Maske zugewandten Seite,
Fig. 26: eine seitliche Ansicht der Maske,
Fig. 27: eine vergrößerte Darstellung gemäß Fig. 26.

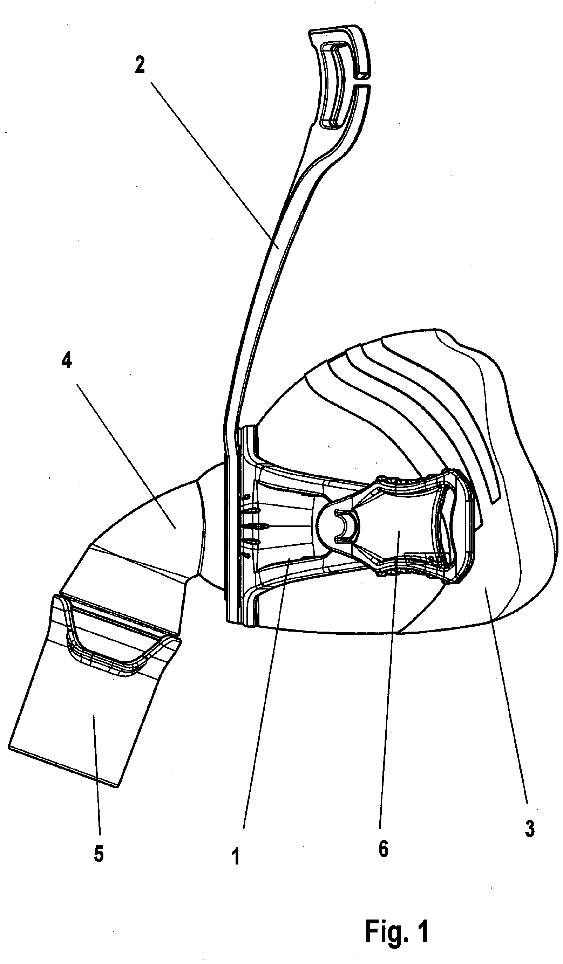

Fig. 1 zeigt eine als Nasalmaske ausgebildete Atemmaske. Die erfindungsgemäße Atemmaske ist modular aufgebaut. Als Basis der Atemmaske dient der Maskenkörper (1). Dieser ist beispielsweise als Spritzgussteil aus Kunststoff relativ fest ausgeführt. An dem Maskenkörper (1) setzen die Stirnstütze (2), die einen sicheren Sitz der Maske auf dem Gesicht eines nicht dargestellten Patienten gewährleistet, und der Maskenwulst (3), der durch seine Passform und seine elastische Beschaffenheit für einen dichten Abschluss der Atemmaske am Patienten sorgt, an. Das Anschlussstück (4) stellt die Verbindung zu einem nicht dargestellten Schlauch dar, über den das Atemgas von einem Beatmungsapparat zur Atemmaske transportiert wird. Der Schlauch wird mittels einer auf dem Anschlussstück (4) drehbeweglich gelagerten Hülse (5) mit dem Anschlussstück (4) verbunden. Mithilfe von seitlich mit dem Maskenkörper (1) verbindbaren Clips (6) kann die Atemmaske durch Bänder sicher am Kopf des Patienten fixiert werden.

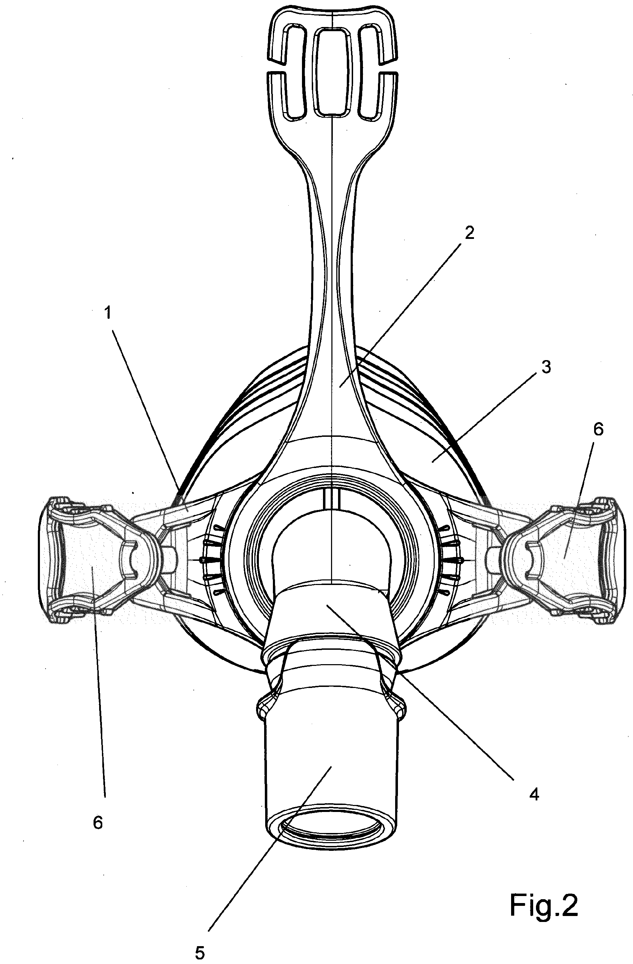

Fig. 2 zeigt die bereits in Fig. 1 dargestellte Ausführungsform einer erfindungsgemäßen Atemmaske von vorn. Zu erkennen ist die Symmetrie des Aufbaus entlang der vertikalen Mittelachse. An beiden Seiten des Maskenkörpers (1) befindet sich hier ein Clip (6) zur Bandaufnahme, jedoch ist auch eine asymmetrische Ausführungsform denkbar. Dies kann beispielsweise umgesetzt werden, indem auf einer Seite die Verbindung von Atemmaske und Halteband mit einem Clip (6) realisiert wird, während auf der anderen Seite eine Schlaufe und ein Haken genutzt werden.

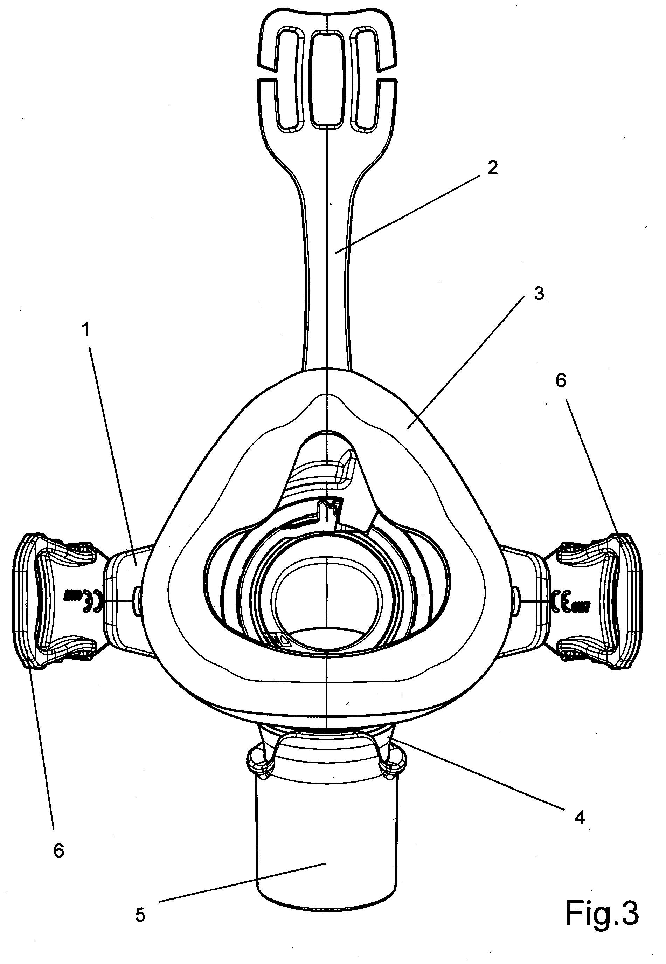

Fig. 3 zeigt die Atemmaske aus Fig. 1 von hinten. Zu sehen ist eine trianguläre Grundform des Maskenwulstes (3) sowie das Innere der Atemmaske durch die patientenseitige Öffnung des Maskenwulstes (3) hindurch. An der oberen Seite des Maskenwulstes (3) ragt die Stirnstütze (2) hervor. An den Seiten der Atemmaske sind die mit dem Maskenkörper (1) verbundenen Clips (6) zur Bandankopplung zu sehen. An der unteren Seite des Maskenwulstes (3) ragt das Anschlussstück (4) mit der montierten Hülse (5) hervor.

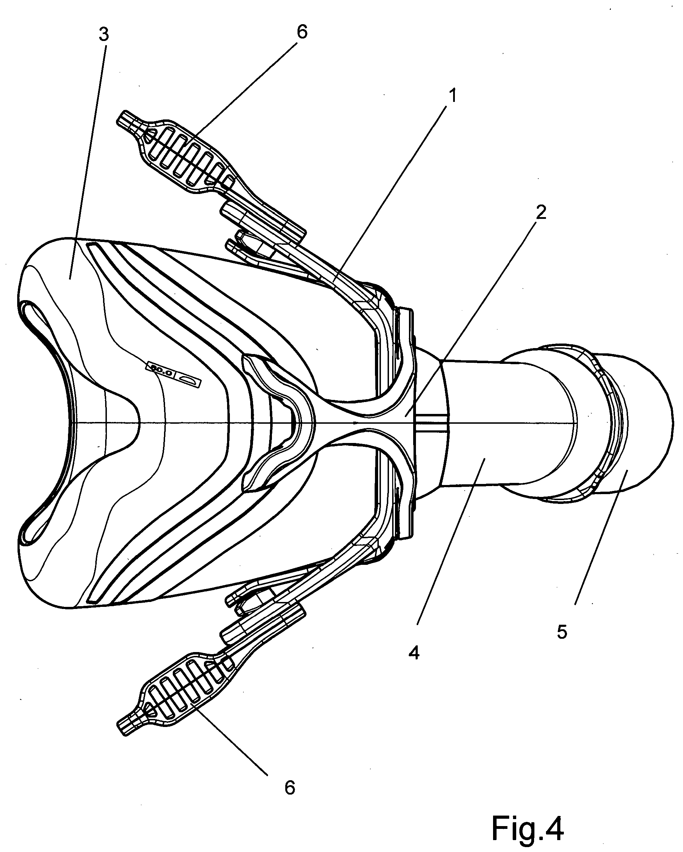

Fig. 4 zeigt die Atemmaske aus Fig. 1 von oben. In dieser Ansicht ist eine der menschlichen Anatomie angepasste Formgebung der Stirnstütze (2) erkennbar. Aus der sich aus der Verbindung mit dem Maskenkörper (1) ergebenden Fläche ragt das Kopfende der Stirnstütze (2) in Richtung eines Patienten, um auch ohne übermäßiges Neigen der gesamten Atemmaske eine ausreichende Stützfunktion an der Stirn eines Patienten zu erreichen.

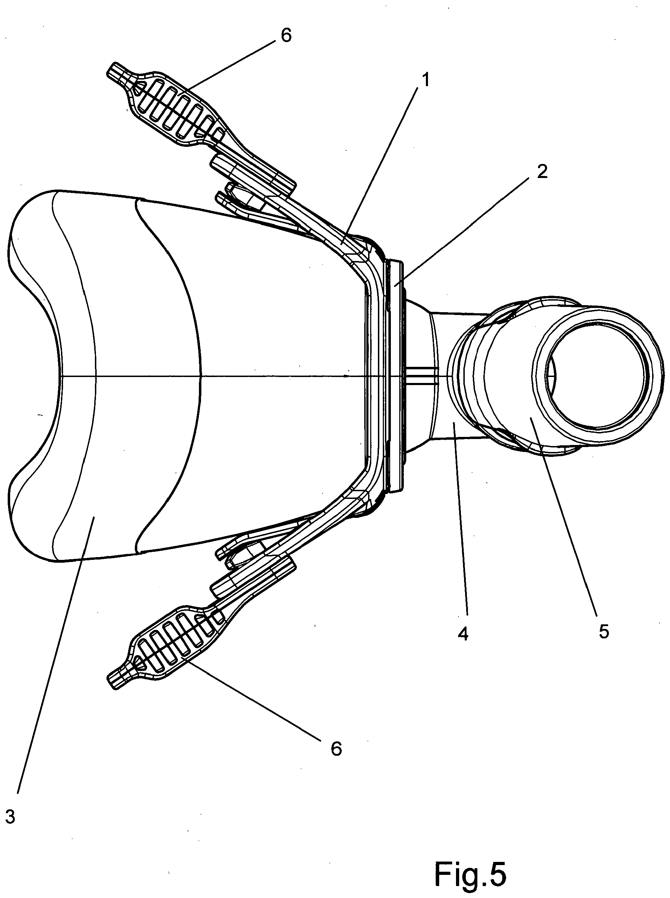

In Fig. 5 ist die in Fig. 1 gezeigte Atemmaske von unten dargestellt. Zu sehen sind der Maskenkörper (1), die Stirnstütze (2), der Maskenwulst (3) auf der einem Patienten zugewandten Seite, das Anschlussstück (4) auf der einem Patienten abgewandten Seite mit montierter Hülse (5) und Clips (6) zur Bandankopplung an die Atemmaske.

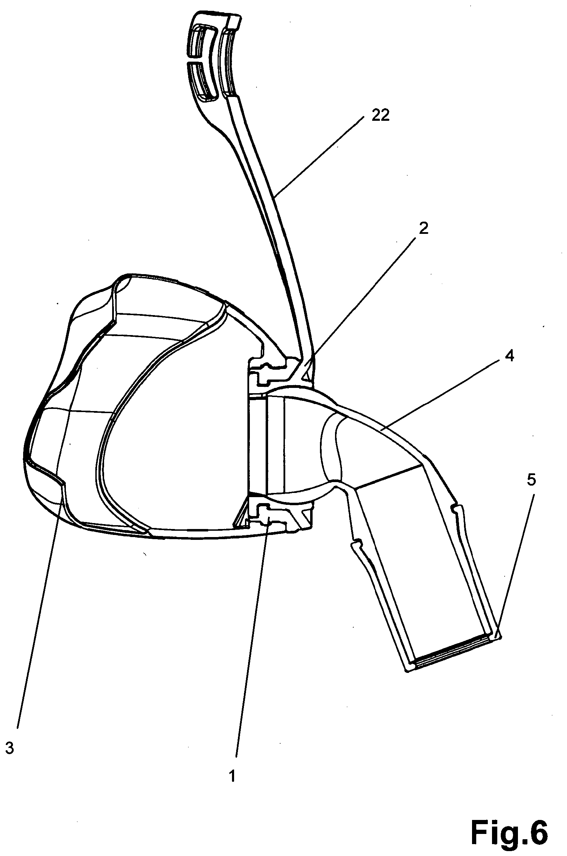

Fig. 6 zeigt einen vertikalen Schnitt durch die Mitte der in Fig. 1 gezeigten Atemmaske. Zu sehen sind der Maskenkörper (1), die Stirnstütze (2), der Maskenwulst (3) auf der einem Patienten zugewandten Seite und das Anschlussstück (4) mit montierter Hülse (5) auf der einem Patienten abgewandten Seite der Atemmaske.

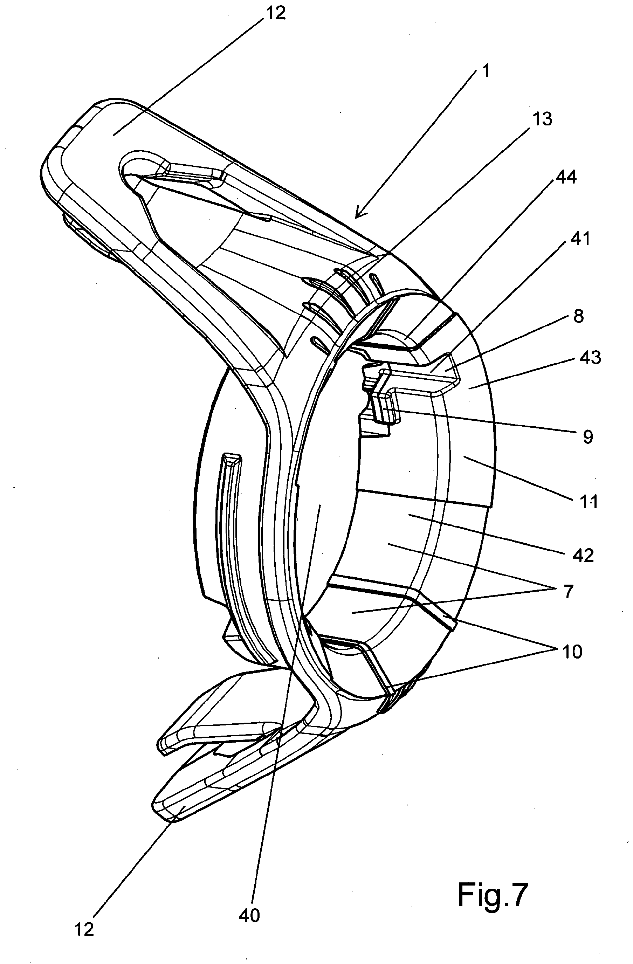

In Fig. 7 wird eine perspektivische Darstellung des Maskenkörpers (1) gezeigt. Zu erkennen ist eine zentrale, runde Öffnung (40) des Maskenkörpers. Die Öffnung (40) dient der Aufnahme der Stirnstütze (2) und bildet eine Schnittstelle (41) zu der Stirnstütze (2).
Die Öffnung (40) des Maskenkörpers (1) wird in einem Bereich durch ein Rohr (42) konstanten Durchmessers definiert. Der rohrförmige Bereich (42) ist mehr als 4 mm lang, bevorzugt mehr als 6 mm lang, besonders bevorzugt mehr als 8 mm lang. Dies ergibt einen langen Strömungsweg für die Atemgase. Der rohrförmige Bereich (42) kickt dann in einem Winkel von 20 - 70°, bevorzugt 25 - 55° relativ zum rohrförmigen Bereich nach außen ab und bildet einen trichterförmigen Bereich (43). Der Übergang zwischen rohrförmigen Bereich (42) und trichterförmigem Bereich (43) wird durch einen Radius (44) gebildet und ist daher nicht abrupt.

Zu erkennen sind mehrere Ausströmflächen (7) auf der innenliegenden Oberfläche Öffnung (40). Die Ausströmflächen (7) können von Distanzelementen (10) wie Rippen unterbrochen sein. Die Distanzelemente (10) erstrecken sich entlang innenliegende Oberfläche des Maskenkörpers (1) von innen nach außen und leiten so die Atemgase vom Patienten weg nach Außen.

Die Distanzelemente (10) erstrecken sich auch entlang des trichterförmigen Bereiches (43).

In der Öffnung (40) befinden sich mindestens zwei Kanäle (8), die als Aussparung im rohrförmigen Bereich und/oder im trichterförmigen Bereich realisiert sind und die sich so bis in den trichterförmigen Bereich erstrecken können. Diese Kanäle (8) fungieren als Aufnahmen für die mechanische Ankopplung der Stirnstütze (2), die beispielsweise als Bajonettverschluss realisiert werden kann. Diese Kanäle (8) fungieren in einer alternativen Ausfühung in doppelter Funktion sowohl als Aufnahmen für die mechanische Ankopplung der Stirnstütze (2), die beispielsweise als Bajonettverschluss realisiert werden kann, als auch als Abströmkanäle für ausgeatmete Atemgase, deren vollständige Kontur gemeinsam von Maskenkörper (1) und Stirnstütze (2) definiert wird. Für den Bajonettverschluss ist im hinteren Bereich neben jedem Kanal (8) jeweils ein Zahn (9) vorgesehen, der radial nach innen aus der inneren Kontur der Öffnung (40) herausragt und der den benötigten mechanischen Widerstand für den Verschluss bietet. Durch die radiale Verrrastung sind Ausströmflächen (7) zwischen Maskenkörper und Stirnstütze (2) lediglich durch die Höhe der Distanzelemente definiert, die radiale Verrastung ändert die Höhe der Ausstömflächen nicht. Bei der Maske der DE 10 2005 041 717 A1 befindet sich ein Bajonett am Sicherungsring und rastet in Fügerichtung, also axial, in dem Maskenkörper ein. Im Stand der Technik kann die axiale Verrastung der Maske dazu führen, dass die Spalthöhe durch die Verrastung beeinflusst wird, wenn in der Serienfertigung Maßabweisungen der axiale Verrastung höhere oder weniger hohe Rastkräfte benötigen.

Des Weiteren sind auf der inneren Oberfläche der Öffnung (40) mehrere Distanzrippen (10) angeordnet, die eine Verbindung von Maskenkörper (1) und Stirnstütze (2) ohne Spiel bzw. unter Vorspannung ermöglichen. Die Distanzrippen (10) sind im Bereich 100 - 500 *µ*m, bevorzugt 160 - 370 *µ*m hoch und sind so bemessen, dass sie einen Spalt für die Atemgase bilden. Die Distanzrippen können auch unterschiedliche Höhen aufweisen oder einen graduellen Anstieg der Höhe. Bevorzugt sind die Distanzrippen dann innen, im zylindrischen Bereich (42) weniger hoch, beispielsweise 100 - 299 *µ*m, und im trichterförmigen Bereich (43) höher, beispielsweise 300 - 500 *µ*m. Dies führt dazu, dass die Atemgase im trichterförmigen Bereich verlangsamt werden und so langsamer von der Maske abströmen.

Im Bereich (11) der Abströmkanäle (8) ist das Material im Ring- und Trichterbereich (42, 43) entsprechend der Höhe der Distanzrippen (10) verstärkt. Die Distanzrippen (10) und die verstärkten Bereiche (11) begrenzen zudem seitlich die Ausströmflächen (7). Die verstärken Bereich (11) verschließen somit einen möglichen Weg für Ausatemgase in Verbindung der Stirnstütze. Die verstärken Bereich sind daher bevorzugt oben und/oder unten angeordnet, also in den Bereichen die in die Augenregion bzw. zum Mund des Anwenders weisen.
Denkbar sind auch zusätzliche Aussparungen im trichterförmigen Bereich (43) der inneren Oberfläche des Maskenkörpers (1), um einen oder mehrere zusätzliche Ausströmkanäle zu formen.

Innerhalb der inneren Kontur der Öffnung (40) ist auch eine ringförmig umlaufende Vertiefung denkbar, die die ausströmenden Atemgase gleichmäßig auf die angelegten Ausströmflächen (7) verteilt. Diese könnte beispielsweise im Übergangsbereich von Rohr- zu Trichterform der innenliegenden Oberfläche des Maskenkörpers (1) realisiert werden.

An seinen Seiten weist der Maskenkörper (1) zwei Flügel (12) auf, die jeweils eine mechanische Struktur zur Aufnahme einer Bandankopplung aufweisen. Die Bandankopplung kann beispielsweise durch einen Clip (6) erfolgen. Im vorderen Bereich des Maskenkörpers (1) ist dieser auf Höhe der Flügel (12) mit Noppen (13) versehen, die eine bessere Griffigkeit bei der Handhabung der Atemmaske ermöglichen. Die Noppen können auch als Strahlen (13) oder pfeilartige Erhebungen ausgebildet sein, die radial von den Ausströmkanäle (8) wegführen und so die Abströmrichtung der Atemgase anzeigen.

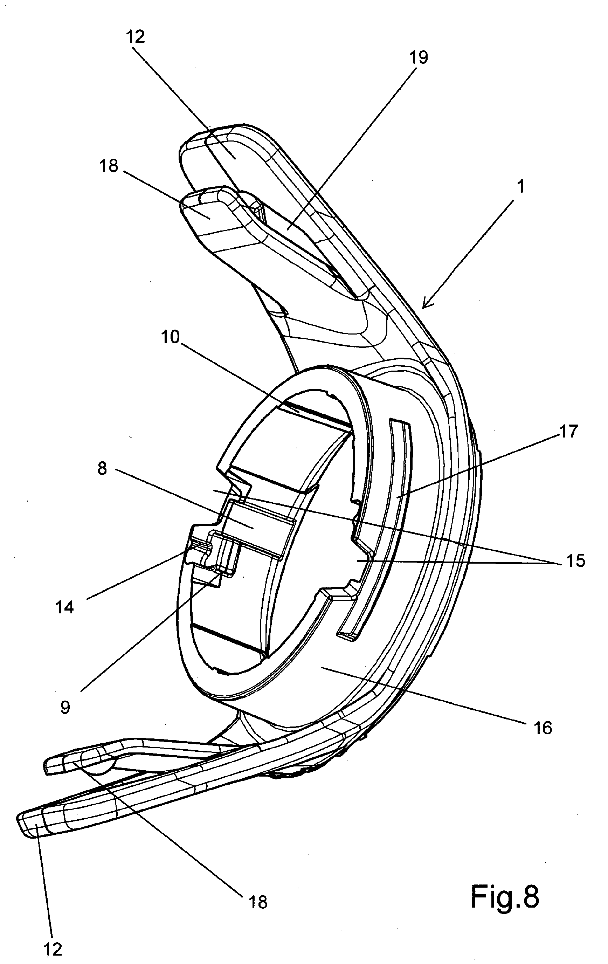

Fig. 8 zeigt eine perspektivische Darstellung des Maskenkörpers (1) von hinten. In dieser Darstellung ist eine Ausführung der im Maskenkörper (1) angelegten Struktur für den Bajonettverschluss zu erkennen. Der Kanal (8) und wird in seiner Grundform hinter dem Zahn (9) zu einer L-förmigen Aussparung ergänzt, was die typische Steck- und Schraubverbindung ermöglicht. Im Konturbereich hinter dem Zahn (9) ist ein Rastzahn (14) vorgesehen, der die Verbindung sichert. Zusätzlich ist in der in Fig. 8 gezeigten Ausführungsform der Atemmaske eine mechanische Kodierung (15) im Maskenkörper (1) derart realisiert, dass der Maskenwulst (3) sich nur in vorgesehenen Positionen mit dem Maskenkörper (1) verbinden lässt. Diese Verbindung erfolgt durch ein Aufstecken des Maskenwulstes (3) auf die äußere Kontur (16) des Maskenkörpers (1). Gesichert wird die Verbindung in der gezeigten Ausführungsform durch mindestens einen Hinterschnitt (17) auf der äußeren Kontur (16) des Maskenkörpers (1). Denkbar sind neben der dargestellten kreisrunden äußeren Kontur (16) auch andersförmige Ausführungen. Insbesondere ist an eine ovale Form der äußeren Kontur (16) gedacht. In Kombination mit der Form der Verbindungsöffnung des Maskenwulstes (3), die mit einem inneren Umfang kleiner oder gleich der äußeren Kontur (16) des Maskenkörpers (1), auch anders geformt sein kann, können die Steifigkeit und das Verhältnis von Höhe und Breite des Maskenwulstes (3) eingestellt werden. Insbesondere ist an eine Kombination von ovaler Ausführung der äußeren Kontur (16) und einer kreisrunden Ausführung der Verbindungsöffnung des Maskenwulstes (3) gedacht. Neben der gezeigten Verbindung von Maskenwulst (3) und Maskenkörper (1) durch einen Hinterschnitt (17), sind auch andere Optionen denkbar. Zum Beispiel ist auch ein Verkleben der Module mit Hilfe eines Zweikomponentenklebstoffs möglich. Die von im Bereich der Flügel (12) bereitgestellte mechanische Aufnahmestruktur für die Bandankopplung besteht im Wesentlichen aus einer Federplatte (18) in Kombination mit einer Aussparung (19) in der Struktur des Flügels (12).

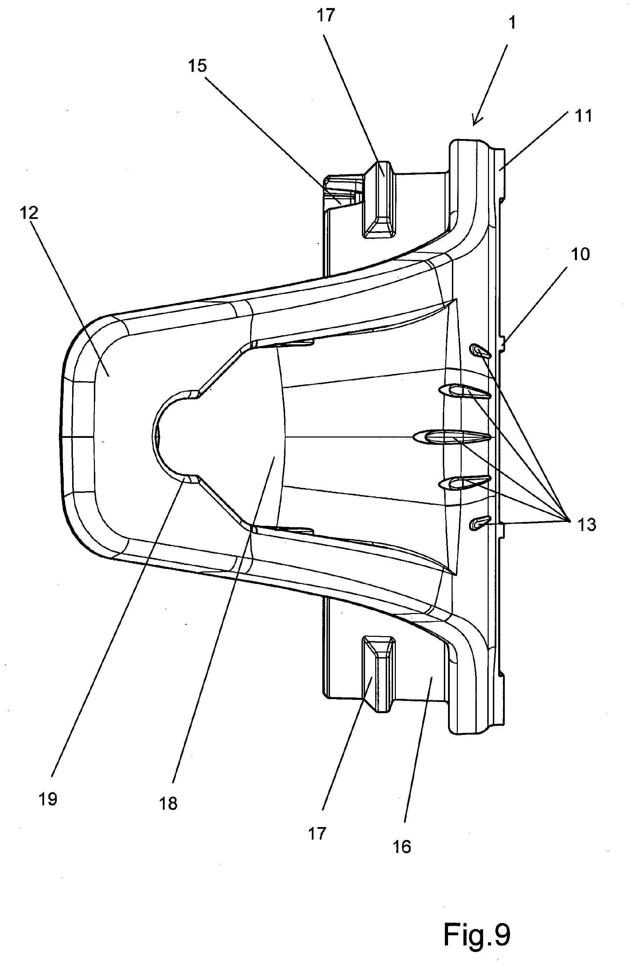

Fig. 9 zeigt eine Seitenansicht des Maskenkörpers (1). Zu sehen ist eine trichterförmige Umsetzung der Aussparung (19) im Flügel (12), die ein benutzerfreundliches Einführen der Bandankopplung, zum Beispiel in Form eines Bänderungsclips (6), ermöglicht. Dies ist erforderlich, da sich dieser Bereich bei Nutzung oder Anlegen der Atemmaske nicht im Sichtfeld des Patienten befindet. Der Trichter (19) führt den Clip (6) automatisch zum Rastpunkt, in dem der Clip (6) durch die Federplatte (18) fixiert wird.

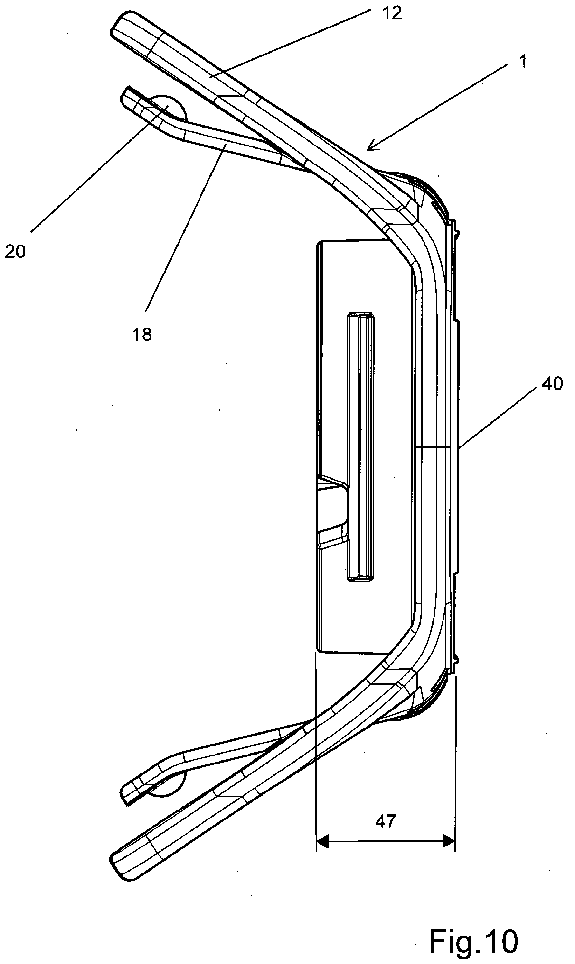

Fig. 10 zeigt eine erfindungsgemäße Ausführungsform des Maskenkörpers (1) von unten. Zu erkennen ist ein Rastzapfen (20) auf der dem Flügel (12) zugewandten Seite der Federplatte (18), der das Einrasten des Bänderungsclips (6) ermöglicht.

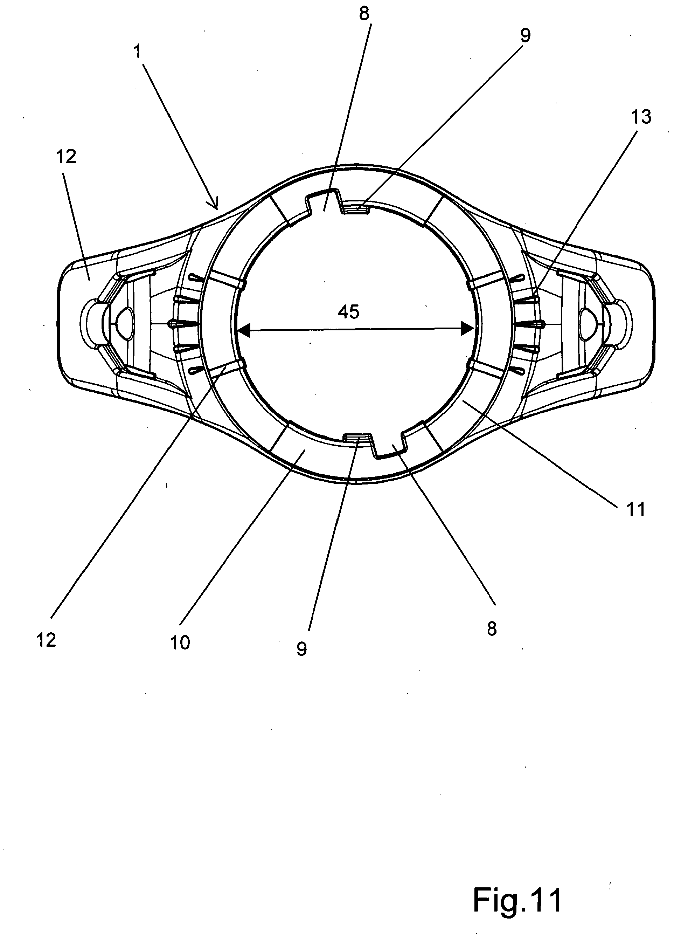

Fig. 11 zeigt den in Fig. 7 dargestellten Maskenkörper (1) in frontaler Ansicht. Zu erkennen ist die Rotationssymmetrie um eine im Mittelpunkt des inneren Radius befindlichen, senkrecht aus der Zeichenebene herausragenden Achse um 180°.

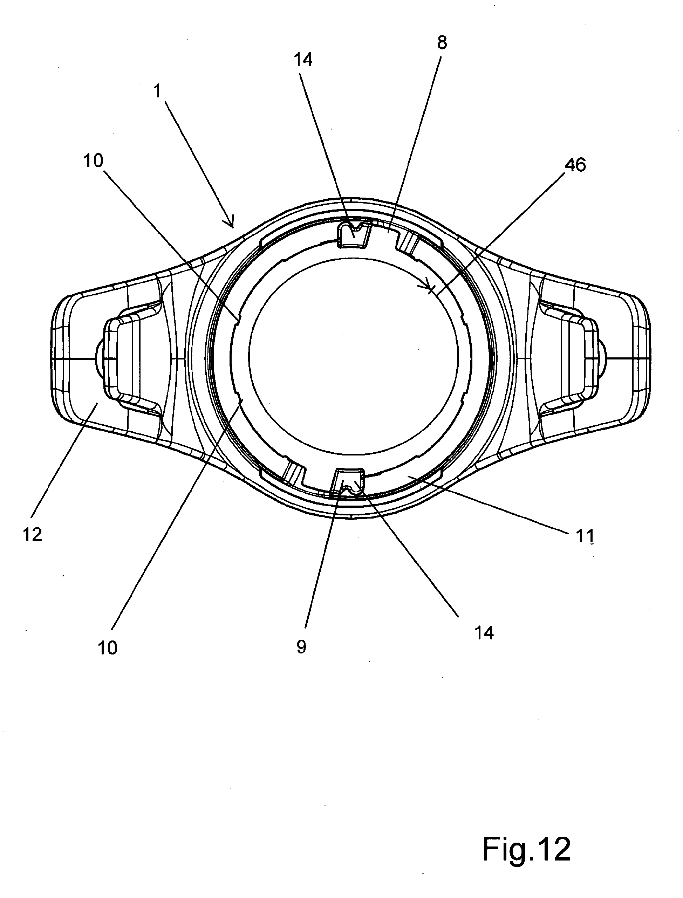

Fig. 12 zeigt den in Fig. 7 dargestellten Maskenkörper (1) von hinten. Ersichtlich sind die radial aus der inneren Kontur (7) herausragenden Distanzrippen (10) und die verstärken Bereiche (11) um die Ausströmkanäle (8). Auch ist die Form der Rastzahns (14) für den Bajonettverschluss zu sehen, der die Bajonettverbindung durch die Verrastung mit dem Gegenstück an der Stirnstütze (2) gegen ungewolltes Verdrehen und Lösen sichert.

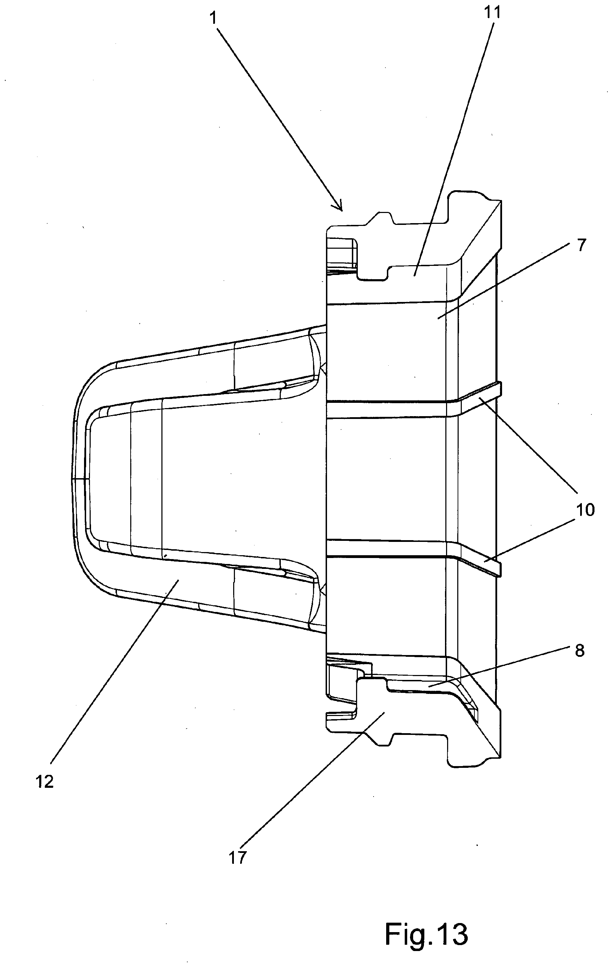

Fig. 13 zeigt einen vertikalen Schnitt durch die Mitte des in Fig. 7 gezeigten Maskenkörpers (1). Zu sehen ist die trichterförmige Öffnung der inneren Kontur des Maskenkörpers (1) zur rechten Seite. Im zwischen den verstärkten Bereichen (11) um die Ausströmkanäle (8) liegenden Bereich der inneren Oberfläche des Maskenkörpers (1), sind jeweils zwei Distanzrippen (10) in gleichen Abständen zueinander und zu den verstärkten Bereichen (11) angeordnet. Die Anzahl und Positionierung der Distanzrippen (10) kann in erfindungsgemäßen Ausführungsformen der Atemmaske variieren.

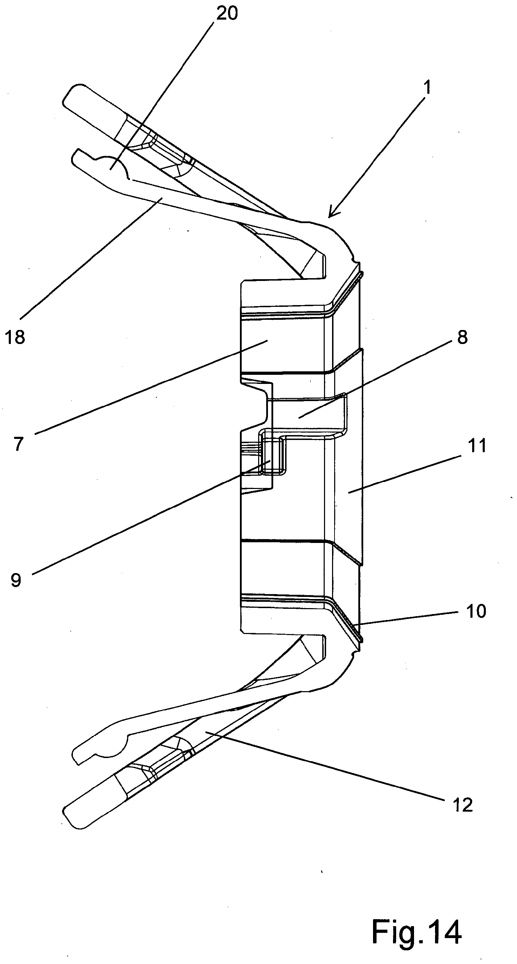

Fig. 14 zeigt einen horizontalen Schnitt durch die Mitte des in Fig. 7 dargestellten Maskenkörpers (1). Zu sehen ist die Positionierung von Federplatte (18) mit Rastzapfen (20) zum Grundkörper des Flügels (12). Der Abstand von Federplatte (18) und Grundkörper des Flügels (12) ist auf einen Clip (6) angepasst.

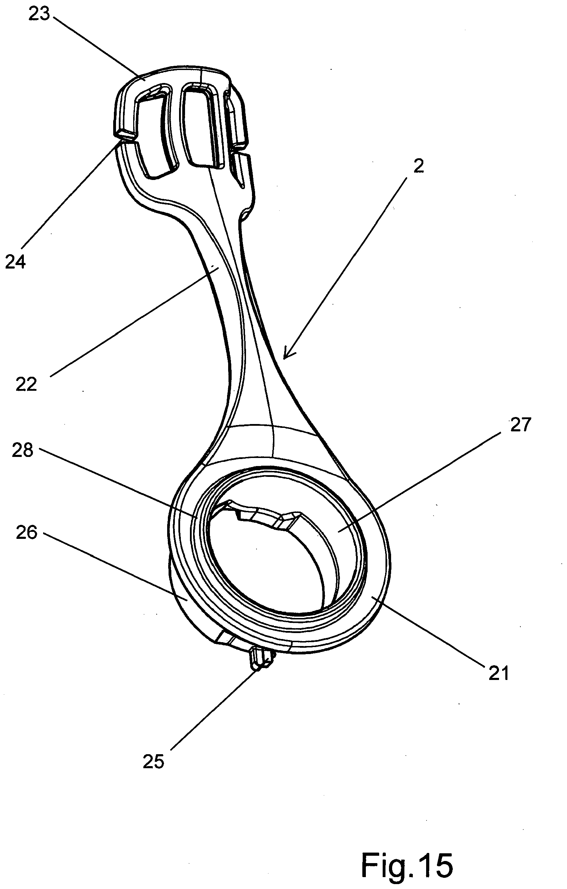

Fig. 15 zeigt eine erfindungsgemäße Ausführungsform einer Stirnstütze (2). Die Basis der Stirnstütze (2) bildet der Stirnstützenverbinder (21), mit dem die Stirnstütze (2) am Maskenkörper (1) befestigt wird. Ein Steg (22) verbindet den Stirnstützenverbinder (21) mit der Bandankopplung (23) am Kopf der Stirnstütze (2). Die Bandankopplung (23) besteht aus drei horizontal nebeneinander angeordneten, in vertikaler Richtung länglich ausgedehnten Aussparungen in der Struktur der Stirnstütze (2). Die beiden äußeren Aussparungen weisen jeweils einen zusätzlichen Spalt (24) in der nach außen gewandten Struktur der Stirnstütze (2) auf. Diese Spalte (24) können zum Einfädeln der Haltebänder auf Stirnhöhe eines Patienten in die Bandankopplung (23) genutzt werden. Alternativ kann das Band auch durch die mittig positionierte Aussparung geführt werden.

Es ist auch an andere Ausführungsformen der Bandankopplung (23) gedacht. Beispielsweise kann diese auch nur durch zwei Aussparungen mit zusätzlichem Spalt (24) umgesetzt werden.
Der Steg (22) ist in seiner Breite in der gezeigten Ausführungsform gegenüber dem Kopfteil mit Bandankopplung (23) und dem Stirnstützenverbinder (21) signifikant reduziert. Dies ermöglicht ein ansprechendes, schlankes Design und die Einsparung von Material.

Der Stirnstützenverbinder (21) bietet zur Vervollständigung des im Maskenkörper (1) angelegten Bajonettverschlusses eine in Positionierung und Anzahl der im Maskenkörper vorhandenen Kanäle (8) entsprechende Anzahl an Verschlusszähnen (25). Diese stehen auf der äußeren Kontur des Verbindungsringes (26) radial nach außen und weisen ihrerseits jeweils einen weiteren radial nach außen gerichteten Zahn auf, der gemeinsam mit dem Rastzahn (14) im Maskenkörper (1) für die Arretierung von Maskenkörper (1) und Stirnstütze (2) in der vorgesehenen Position sorgt. Die innere Kontur des Stirnstützenverbinders (21) ist als Kugelkäfig (27) ausgeführt, der das Anschlussstück (4) beweglich lagern kann. Zum Anschlussstück (4) hin wird der Kugelkäfig (27) durch eine sich nach außen verengende Ringstruktur (28) begrenzt.

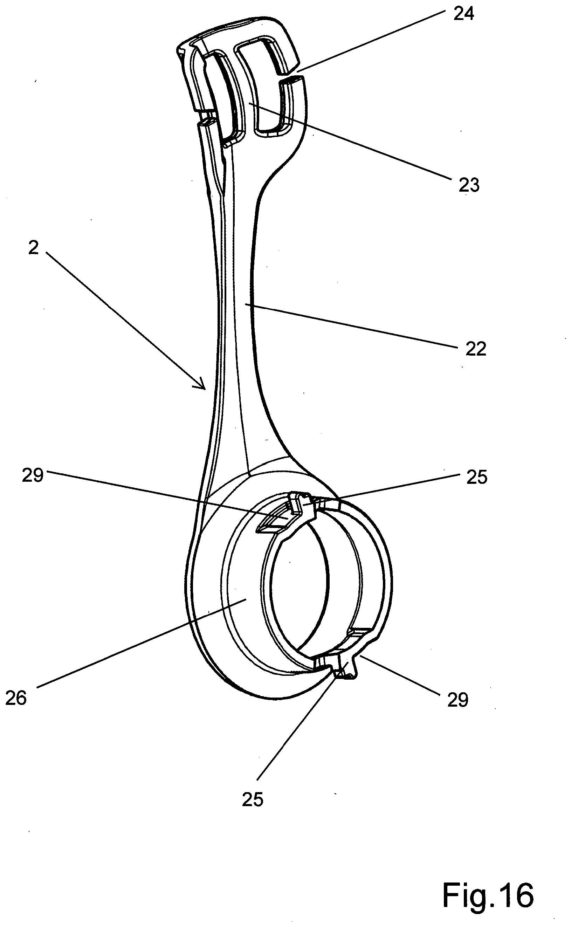

Fig. 16 zeigt eine perspektivische Darstellung einer Stirnstütze (2) von hinten. Die dargestellte Stirnstütze (2) weist zwei Verschlusszähne (25) auf. Jedoch sind auch mehr Verschlusszähne (25) passend zum jeweiligen Maskenkörper (1) denkbar. Erkennbar sind Aussparungen (29) in der äußeren Kontur des Verbindungsringes (26) neben den Verschlusszähnen (25). Diese Aussparungen (29) dienen als zusätzliche Ausströmkanäle und leiten die ausgeatmeten Gase um den jeweiligen Verschlusszahn (25) zum entsprechenden im Maskenkörper (1) angeordneten Kanal (8). Zusätzlich ist auch die Nutzung einer solchen Aussparung (29) als mechanische Kodierung für eine definierte Ausrichtung von Maskenkörper (1), Stirnstütze (2) und Maskenwulst (3) denkbar. Dafür muss die Struktur des Maskenkörpers (1) im Bereich seiner mechanischen Kodierung (15) entsprechend durchlässig sein.

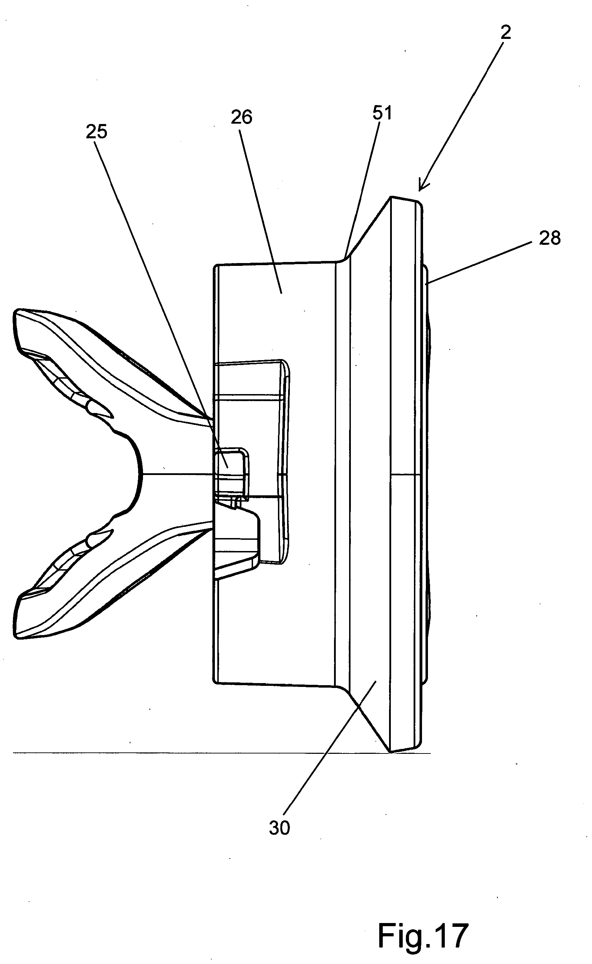

In Fig. 17 ist die Stirnstütze (2) aus Fig. 15 von unten dargestellt. Zu erkennen ist die der inneren Kontur (40, 42, 43) des Maskenkörpers (1) angepasste äußere Kontur (50) des Stirnstützenverbinders (21), die sich ebenfalls aus einem Ring (26) und einem Trichter (30) zusammensetzt. Der Übergang von Ring zum Trichter ist bevorzugt als Radius (51) ausgebildet. Diese Kontur bildet die stirnstützenseitige Abströmoberfläche (30) und definiert gemeinsam mit der innenliegenden Kontur (40) des Maskenkörpers (1) die Form der Abströmkanäle (8) und der Ausatemspalte.

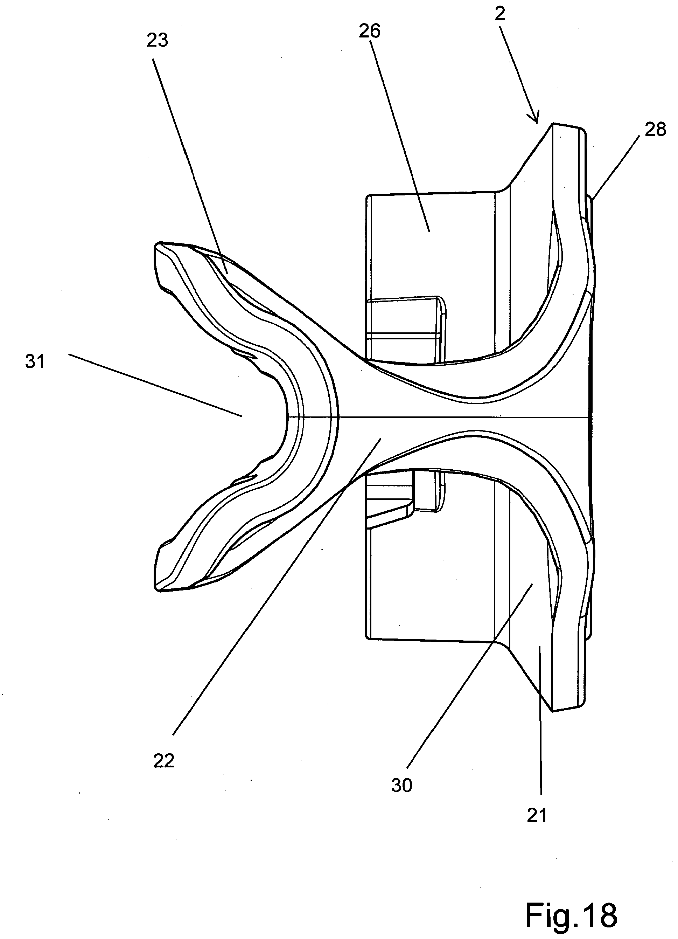

Fig. 18 zeigt die Stirnstütze (2) aus Fig. 15 von oben. Zu erkennen ist, dass das Kopfteil der Stirnstütze (2) in Relation zum Stirnstützenverbinder (21) zu einem Patienten hin ragt. Die Form des die Bandankopplung (23) beherbergenden Kopfes der Stirnstütze (2) weist eine Aussparung (31) auf, die zwischen der Stirn eines Patienten und der Bandankopplung (23) in der Stirnstütze (2) einen Raum bietet, in dem die Bänder liegen können.

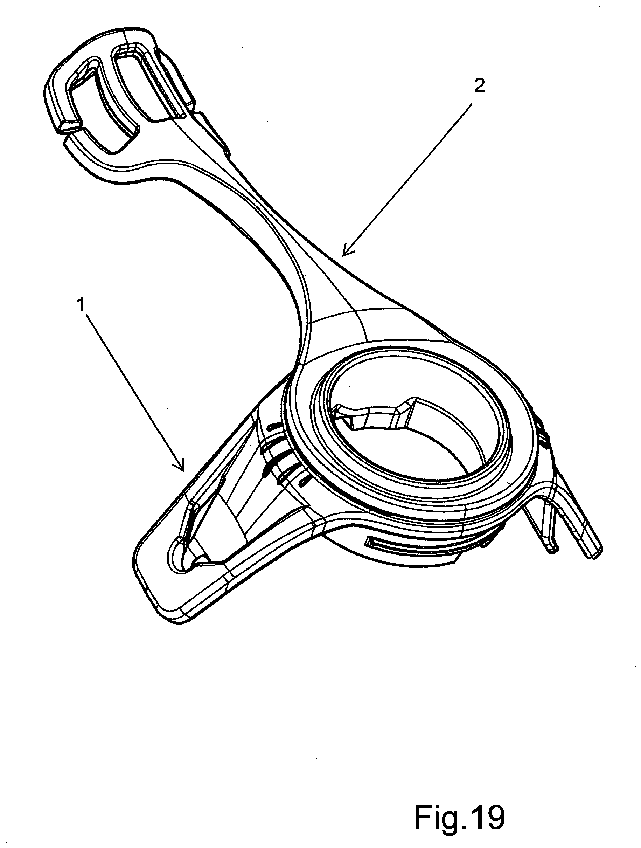

In Fig. 19 sind Maskenkörper (1) und Stirnstütze (2) als montiertes Modul in perspektivischer Darstellung zu sehen. Bei einer rotationssymmetrischen Ausführung des Maskenkörpers (1) kann dieser in genau zwei um 180° verschobenen Positionen mit der Stirnstütze (2) verbunden werden, die funktional identisch sind. Damit lässt sich eine Vereinfachung der Montage der Module der Atemmaske erreichen.

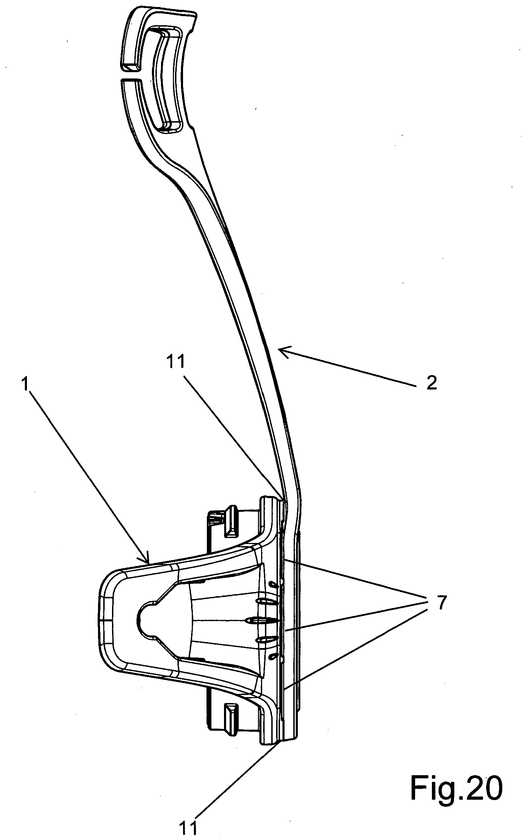

Fig. 20 zeigt eine seitliche Ansicht des montierten Moduls aus Maskenkörper (1) und Stirnstütze (2). Die zwischen den Abströmoberflächen (7) des Maskenkörpers (1), sowie den Ausströmkanälen (8) und der Abströmoberfläche (30) des Stirnstützenverbinders (21) verbleibenden Spalte, dienen als Leitstruktur für die ausgeatmete Luft. Durch die erhöhte Struktur im Bereich (11) der Kanäle (8) im Maskenkörper (1) wird an der oberen Seite in Richtung der Stirnstütze (2) eine Abdichtung des Ausatemspaltes in einem definierten Bereich erreicht. Dies dient der Vermeidung der Ausströmung von Luft in für einen Patienten unangenehme Bereiche, insbesondere die Augen.

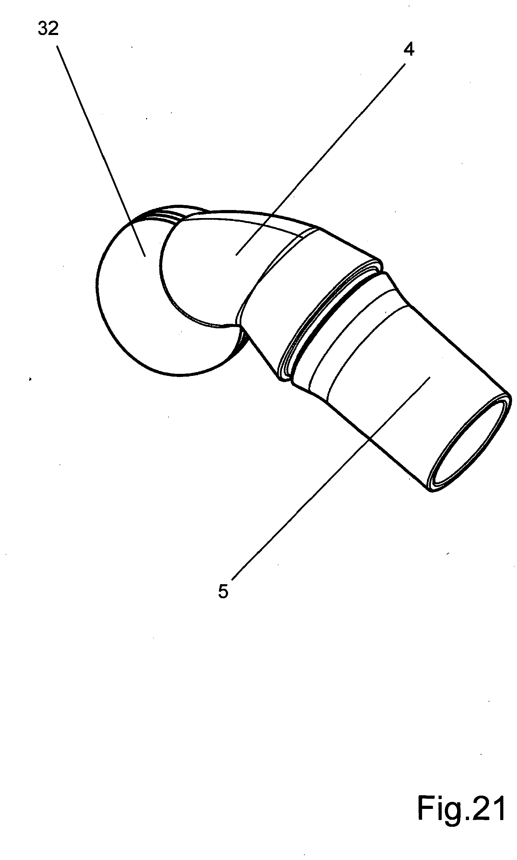

Fig. 21 zeigt eine perspektivische Darstellung eines erfindungsgemäßen Anschlussstückes (4). Das Anschlussstück (4) ist als abgewinkelte Rohrstruktur ausgeführt und weist an einem Ende eine Oberflächenkontur (32) auf, die einer Teilkugel entspricht. Diese Teilkugel (32) dient der beweglichen Verbindung von Anschlussstück (4) und Stirnstütze (2) mittels des im Stirnstützenverbinder (21) realisierten Kugelkäfigs (27).

Am anderen Ende ist eine drehbar gelagerte Hülse (5) als Anschluss für einen nicht dargestellten Schlauch montiert.

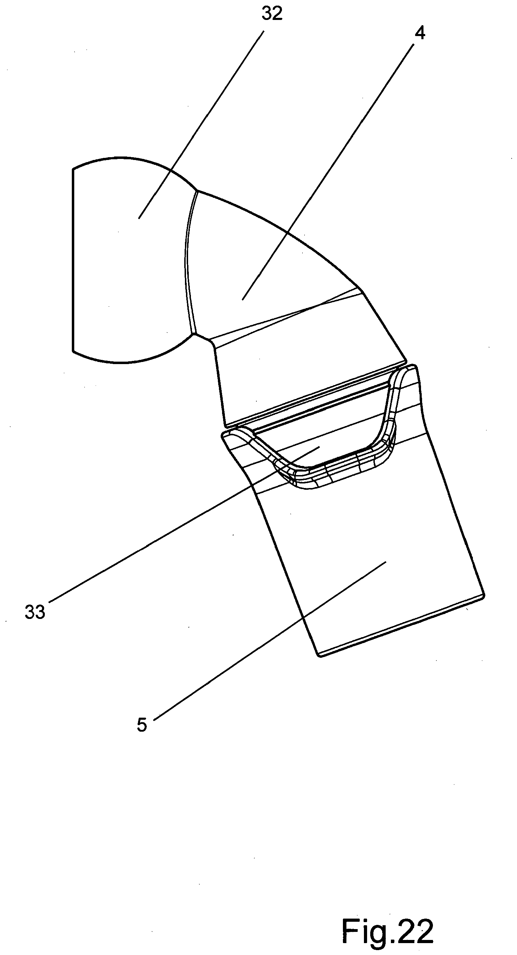

Fig. 22 zeigt eine Seitenansicht eines erfindungsgemäßen Anschlussstückes (4) mit einer alternativen drehbar gelagerten Hülse (5). Die Hülse (5) verdickt sich am zur Atemmaske orientierten Ende und weist an diesem Ende Aussparungen (33) auf.

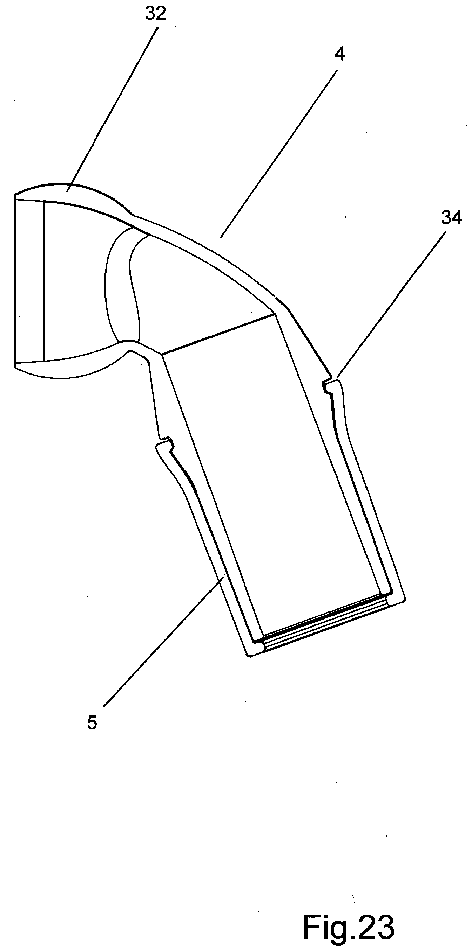

Fig. 23 zeigt einen vertikalen Schnitt durch das Anschlussstück (4). Zu sehen sind am oberen Ende die Oberflächenkontur in Form einer Teilkugel (32), die rohrförmige innere Struktur und eine Verdickung der Struktur hin zu einer ringförmig umlaufenden Nut (34), die die Hülse mittels einem an dieser vorhandenen Hinterschnitt drehbar lagert.

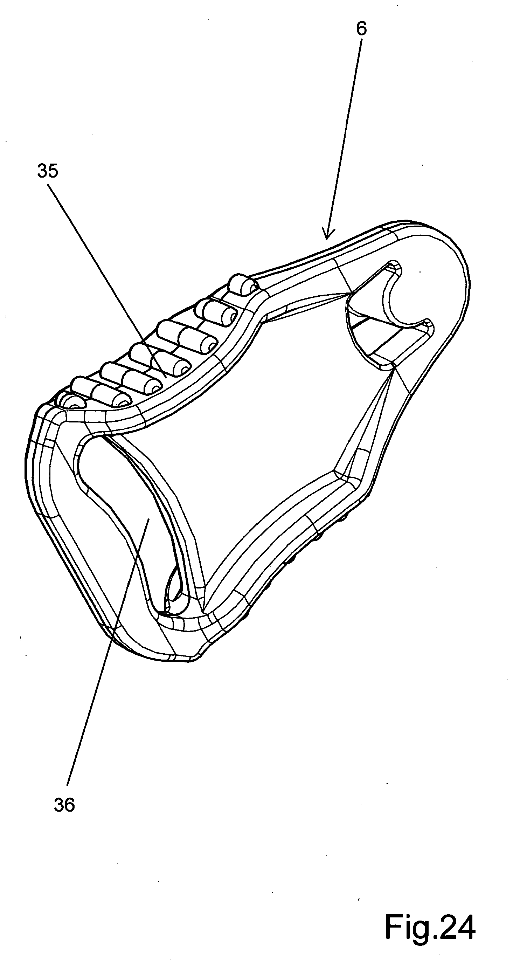

Fig. 24 zeigt eine perspektivische Darstellung eines Bänderungsclips (6). An seiner Ober- und Unterseite weist der Clip (6) Vertiefungen auf, die mit Noppen (35) besetzt sind. Diese dienen einer verbesserten Griffigkeit. An einem Ende weist der Clip (6) eine Öse (36) auf, durch die ein Halteband gezogen und so befestigt werden kann.

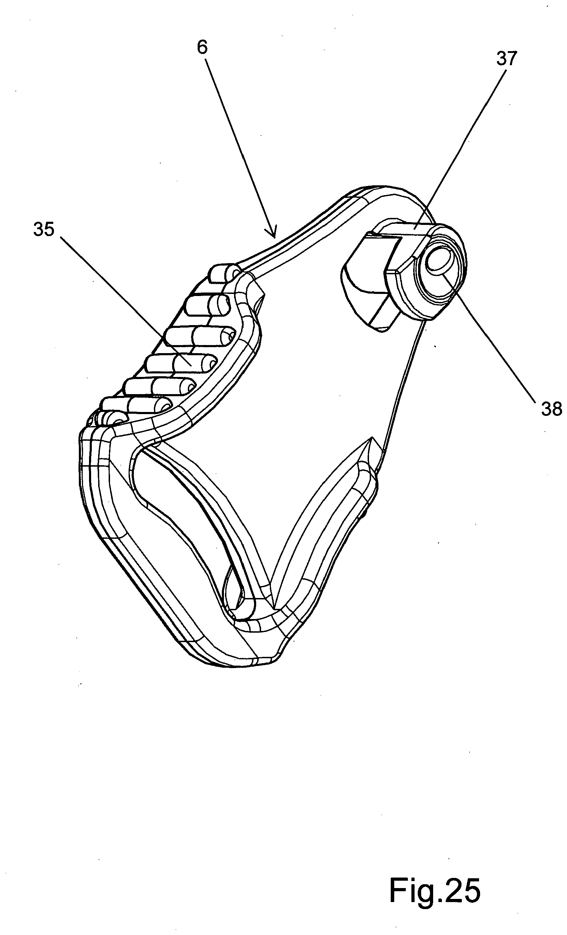

Fig. 25 zeigt eine perspektivische Darstellung des Bänderungsclips (6) von der im montierten Zustand zur Atemmaske hin orientierten Seite. Erkennbar sind ein Zapfen (37) mit pilzförmigem Hinterschnitt, sowie eine als Pfanne ausgeführte Vertiefung (38) im Kopf des Zapfens (37). Diese Vertiefung (38) sorgt im Zusammenspiel mit dem Rastzapfen (20) auf der Federplatte (18) des Maskenkörpers (1) für das Einrasten des Bänderungsclips (6) in der vorgesehenen Position am Flügel (12) des Maskenkörpers (1).

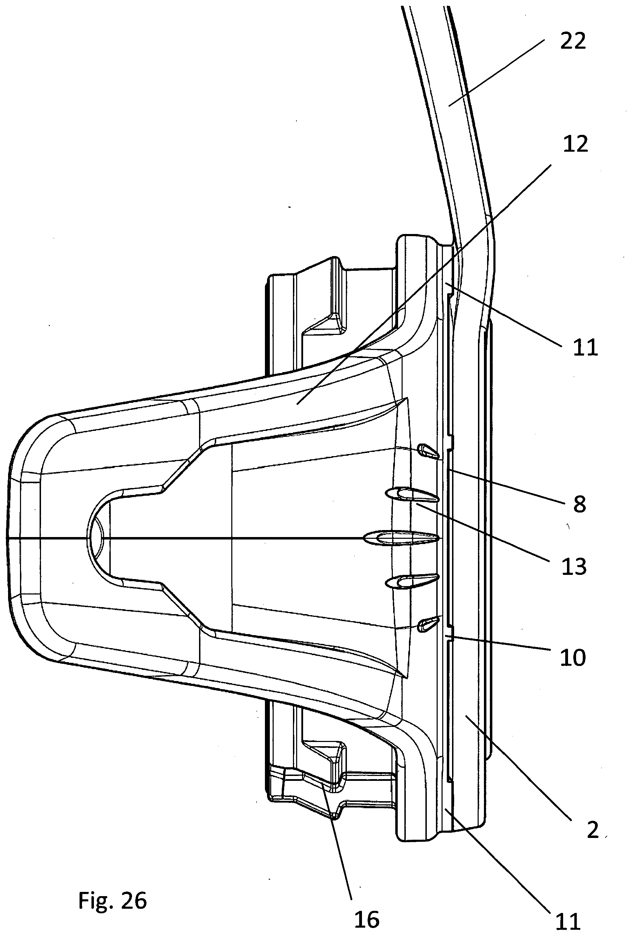

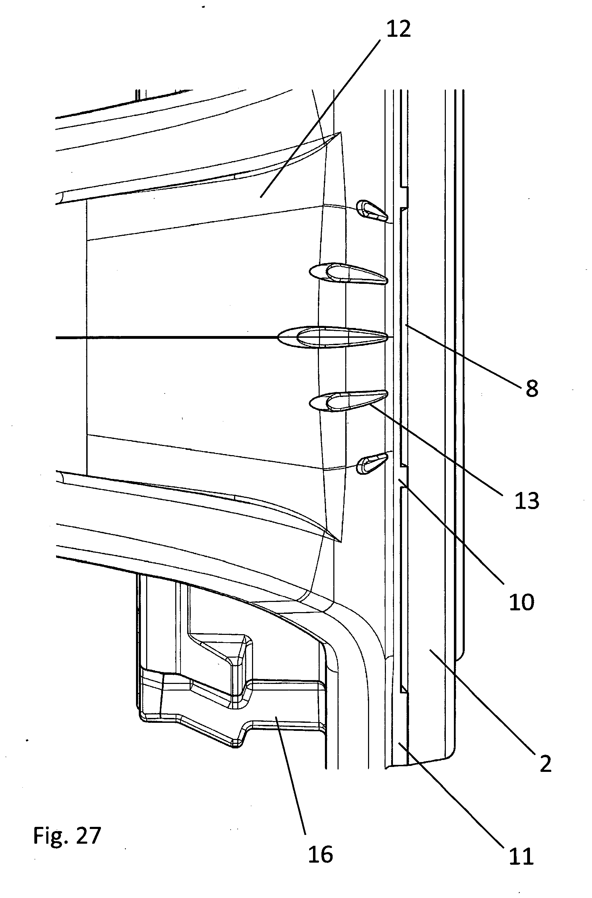

In Fig. 26 und Fig. 27 wird der Maskenkörpers (1) mit der Stirnstütze (2) gezeigt. Zu erkennen ist der seitliche Flügel (12) des Maskenkörpers mit der mechanischen Struktur zur Aufnahme einer Bandankopplung. Die Bandankopplung kann beispielsweise durch einen Clip (6) erfolgen. Im vorderen Bereich des Maskenkörpers (1) ist dieser auf Höhe der Flügel (12) mit Strukturen (13) versehen, die eine bessere Griffigkeit bei der Handhabung der Atemmaske ermöglichen. Die Strukturen können auch als Strahlen (13) oder pfeilartige Erhebungen ausgebildet sein, die radial von den Ausströmkanäle (8) wegführen und so die Abströmrichtung der Atemgase anzeigen.

Der Maskenkörpers (1) weist Distanzelemente (10) und verstärken Bereiche (11) auf, die an die Kontaktstelle zwischen Maskenkörpers (1) und Stirnstütze (2) darstellen. Da die Distanzelemente (10) punktartig oder linear oder segmentweise ausgeführt sind ergeben sich an der Kontaktstelle zwischen Maskenkörpers (1) und Stirnstütze Kanäle (8) für das ausgeatmete Atemgas. Die vollständige Kontur der Kanäle wird gemeinsam von Maskenkörper (1) und Stirnstütze (2) mit Distanzelementen (10) und verstärken Bereiche (11) definiert. Dabei ist erfindungsgemäß auch vorgesehen die Distanzelementen (10) und verstärken Bereiche (11) an der Stirnstütze (2) vorgesehen sein können.

Die Distanzrippen (10) ermöglichen eine Verbindung von Maskenkörper (1) und Stirnstütze (2) ohne Spiel bzw. unter Vorspannung. Die Distanzrippen (10) sind im Bereich 100 - 500 *µ*m, bevorzugt 160 - 370 *µ*m hoch und sind so bemessen, dass sie einen Spalt für die Atemgase bilden.

Im Bereich (11) der Abströmkanäle (8) ist das Material im Ring- und Trichterbereich (42, 43) entsprechend der Höhe der Distanzrippen (10) verstärkt. Die Distanzrippen (10) und die verstärkten Bereiche (11) begrenzen zudem seitlich die Ausströmflächen (7). Die verstärken Bereich (11) verschließen somit einen möglichen Weg für Ausatemgase in Verbindung der Stirnstütze. Die verstärken Bereich sind daher bevorzugt oben benachbart zum Steg (22) der und/oder unten gegenüber dem Steg (22) angeordnet, also in den Bereichen die in die Augenregion bzw. zum Mund des Anwenders weisen.

Eine mechanische Sicherung am Maskenkörper (1) ist derart realisiert, dass der Maskenwulst sich gesichert,mit dem Maskenkörper (1) verbinden lässt. Diese Verbindung erfolgt durch ein Aufstecken des Maskenwulstes auf die äußere Kontur (16) des Maskenkörpers (1). Gesichert wird die Verbindung in der gezeigten Ausführungsform durch mindestens einen Hinterschnitt auf der äußeren Kontur (16) des Maskenkörpers (1).

## Patentansprüche

1. Atemmaske mit einem Maskenkörper (1) und einer Öffnung (40) für Atemgase, und einem Verbinder (21) mit einer äußeren Kontur (26) der zumindest mit der äußeren Kontur (26) teilweise in die Öffnung (40) eingeführt ist und mechanisch und lösbar in der Öffnung gehalten wird, wobei ein Anschlußstück (4) für die Zuleitung von Atemgas lösbar mit dem Verbinder (21) verbunden ist, wobei Ausströmflächen (7) für Ausatemgase in der Schnittstelle zwischen der innenliegenden Oberfläche der Öffnung (40) und der äußeren Kontur (26) des Verbinders (21) und Distanzelementen (10) zwischen der innenliegenden Oberfläche der Öffnung (40) und der äußeren Kontur (26) des Verbinders (21), die die Höhe von Dimensionierung der Ausströmflächen (7) vorgeben, **dadurch gekennzeichnet, dass** der Verbinder (21) ein integraler Teil der Stirnstütze (2) ist und deren Maskenkörper (1) eine zentrale, runde Öffnung (40) aufweist, die der Aufnahme der Stirnstütze (2) dient und so eine Schnittstelle (41) zu der Stirnstütze (2) bildet.

2. Atemmaske nach zumindest einem der vorhergehenden Ansprüche, deren Öffnung (40) rund ist und einen inneren Durchmesser (45) von zumindest 1 cm aufweist und/oder deren Öffnung (40) eine Länge (47) von zumindest von 4 mm aufweist

3. Atemmaske nach zumindest einem der vorhergehenden Ansprüche, deren Öffnung (40) des Maskenkörpers (1) in einem Bereich durch ein Rohr (42) konstanten Durchmessers definiert wird und deren rohrförmiger Bereich (42) mehr als 4 mm lang ist.

4. Atemmaske nach zumindest einem der vorhergehenden Ansprüche, deren rohrförmiger Bereich (42) in einem Winkel von 20 - 90° relativ zum rohrförmigen Bereich nach außen abknickt und einen trichterförmigen Bereich (43) bildet.

5. Atemmaske nach zumindest einem der vorhergehenden Ansprüche, die mehrere Ausströmflächen (7) auf der innenliegenden Oberfläche der Öffnung (40) aufweist, die von Distanzelementen (10), wie Rippen, unterbrochen sind und deren Distanzelemente (10) sich entlang innenliegende Oberfläche des Maskenkörpers (1) von innen nach außen erstrecken und so die Atemgase vom Patienten weg nach Außen leiten.

6. Atemmaske nach zumindest einem der vorhergehenden Ansprüche, deren Stirnstütze (2) als Einheit mit einer Gelenkaufnahme (27) für ein Anschlussstück (4) ausgeführt ist.

7. Atemmaske nach zumindest einem der vorhergehenden Ansprüche, deren Ausströmstruktur für ausgeatmete Gase durch die Oberflächen der Öffnung (40) des Maskenkörpers (1), und der Stirnstütze (2) definiert ist.

8. Atemmaske nach zumindest einem der vorhergehenden Ansprüche, bei der im Umfang der Öffnung (40) mindestens zwei Kanäle (8) angeordnet sind, die als Aussparung im rohrförmigen Bereich und/oder im trichterförmigen Bereich realisiert sind und bei der die Kanäle (8) als Aufnahmen für die mechanische Ankopplung der Stirnstütze (2) fungieren, die beispielsweise als Bajonettverschluss realisiert werden kann, wobei die Kanäle (8) in doppelter Funktion sowohl als Aufnahmen für die mechanische Ankopplung der Stirnstütze (2), als auch als Abströmkanäle für ausgeatmete Atemgase dienen.

9. Atemmaske nach zumindest einem der vorhergehenden Ansprüche, bei der die Kanäle (8) als Aufnahmen für die mechanische Ankopplung der Stirnstütze (2) fungieren, die als Bajonettverschluss realisiert ist und bei der für den Bajonettverschluss im hinteren Bereich neben jedem Kanal (8) jeweils ein Zahn (9) vorgesehen ist, der radial nach innen aus der inneren Kontur der Öffnung (40) herausragt und der den benötigten mechanischen Widerstand für den Verschluss bietet.

10. Atemmaske nach zumindest einem der vorhergehenden Ansprüche, bei der die mechanische Verrastung zwischen Maskenkörper und Stirnstütze in radialer Richtung durch radial zur inneren Kontur der Öffnung (40) angeordnete Rastelemente erfolgt.

11. Atemmaske nach zumindest einem der vorhergehenden Ansprüche, bei der auf der inneren Oberfläche der Öffnung (40) mehrere Distanzrippen (10) angeordnet sind, die eine Verbindung von Maskenkörper (1) und Stirnstütze (2) ohne Spiel bzw. unter Vorspannung ermöglichen.

12. Atemmaske nach zumindest einem der vorhergehenden Ansprüche, bei der die Distanzrippen (10) im Bereich 100 - 500 *µ*m hoch sind und so bemessen sind, dass sie einen Spalt für die Atemgase bilden oder bei der die Distanzrippen (10) unterschiedliche Höhen aufweisen oder einen graduellen Anstieg der Höhe oder bei der die Distanzrippen (10) innen, im rohrförmigen Bereich (42) weniger hoch sind, beispielsweise 100 - 299 *µ*m, und im trichterförmigen Bereich (43) höher, beispielsweise 300 - 500 *µ*m.

13. Atemmaske nach zumindest einem der vorhergehenden Ansprüche, bei der im Bereich (11) der Abströmkanäle (8) das Material im Ring- und Trichterbereich (42, 43) entsprechend der Höhe der Distanzrippen (10) verstärkt ist und bei der die Distanzrippen (10) und die verstärkten Bereiche (11) seitlich die Ausströmflächen (7) begrenzen.

14. Atemmaske nach zumindest einem der vorhergehenden Ansprüche, bei der im Maskenkörper sichtbare und/oder fühlbare Strukturen (13) ausgebildet sein, die radial von den Ausströmkanälen wegführen und so die Abströmrichtung der Atemgase anzeigen.

## Claims

1. A breathing mask having a mask body (1) and an opening (40) for breathing gases, and a connector (21) having an outer contour (26), which is at least partially introduced with the outer contour (26) into the opening (40) and is held mechanically and detachably in the opening, wherein an adapter (4) for supplying breathing gas is detachably connected to the connector (21), wherein discharge faces (7) for exhalation gases in the interface between the internal surface of the opening (40) and the outer contour (26) of the connector (21) and spacer elements (10) between the internal surface of the opening (40) and the outer contour (26) of the connector (21), which predefine the height of dimensioning of the discharge faces (7), **characterized in that** the connector (21) is an integral part of the forehead support (2) and the mask body (1) of which has a central, round opening (40) which serves to receive the forehead support (2) and thus forms an interface (41) with the forehead support (2).

2. The breathing mask according to at least one of the preceding claims, the opening (40) of which is round and has an inner diameter (45) of at least 1 cm and/or the opening (40) of which has a length (47) of at least 4 mm.

3. The breathing mask according to at least one of the preceding claims, the opening (40) of which of the mask body (1) is defined in a region by a tube (42) having a constant diameter and the tubular region (42) of which is more than 4 mm long.

4. The breathing mask according to at least one of the preceding claims, the tubular region (42) of which kinks outwardly at an angle of 20 - 90° relative to the tubular region and forms a funnel-shaped region (43).

5. The breathing mask according to at least one of the preceding claims, which has multiple discharge faces (7) on the internal surface of the opening (40), which are interrupted by spacer elements (10) such as ribs, and the spacer elements (10) of which extend along the internal surface of the mask body (1) from the inside out and thus conduct the breathing gases away from the patient to the outside.

6. The breathing mask according to at least one of the preceding claims, the forehead support (2) of which is designed as a unit having an articulated receptacle (27) for an adapter (4).

7. The breathing mask according to at least one of the preceding claims, the discharge structure of which for exhaled gases is defined by the surfaces of the opening (40) of the mask body (1), and of the forehead support (2).

8. The breathing mask according to at least one of the preceding claims, in which at least two channels (8) are arranged in the circumference of the opening (40), which channels are realized as a recess in the tubular region and/or in the funnel-shaped region and in which the channels (8) function as receptacles for the mechanical coupling of the forehead support (2) which can be realized, for example, as a bayonet fastening, wherein the channels (8), having a dual function, serve both as receptacles for the mechanical coupling of the forehead support (2) and as escape channels for exhaled breathing gases.

9. The breathing mask according to at least one of the preceding claims, in which the channels (8) function as receptacles for the mechanical coupling of the forehead support (2) which is realized as a bayonet fastening and in which a tooth (9) is provided in each case for the bayonet fastening in the rear region next to each channel (8), which tooth projects radially inwards from the inner contour of the opening (40) and which offers the required mechanical resistance for the fastening.

10. The breathing mask according to at least one of the preceding claims, in which the mechanical catching is effected between the mask body and the forehead support in the radial direction by latching elements arranged radially to the inner contour of the opening (40).

11. The breathing mask according to at least one of the preceding claims, in which multiple spacer ribs (10) are arranged on the inner surface of the opening (40), which make possible a connection of the mask body (1) and forehead support (2) without play or respectively under prestress.

12. The breathing mask according to at least one of the preceding claims, in which the spacer ribs (10) are in the range of 100 - 500 *µ*m high and are dimensioned such that they form a gap for the breathing gases or in which the spacer ribs (10) have different heights or a gradual increase in the height or in which the spacer ribs (10) are less high, for example 100 - 299 *µ*m, internally, in the tubular region (42) and higher, for example 300 - 500 *µ*m, in the funnel-shaped region (43).

13. The breathing mask according to at least one of the preceding claims, in which in the region (11) of the escape channels (8) the material in the ring and funnel region (42, 43) is reinforced in accordance with the height of the spacer ribs (10) and in which the spacer ribs (10) and the reinforced regions (11) laterally delimit the discharge faces (7).

14. The breathing mask according to at least one of the preceding claims, in which visible and/or feelable structures (13) are configured in the mask body, which lead away radially from the discharge channels and thus indicate the escape direction of the breathing gases.

## Revendications

1. Masque respiratoire doté d'une coque de masque (1) et d'une ouverture (40) pour les gaz respiratoires, ainsi que d'un raccord (21) avec un contour extérieur (26), qui est introduit au moins en partie par le contour extérieur (26) dans l'ouverture (40) et est maintenu mécaniquement dans l'ouverture de façon amovible, dans lequel une pièce de raccordement (4) de la conduite d'amenée de gaz respiratoire est reliée au raccord (21) de façon amovible, dans lequel les surfaces d'exhalation (7) des gaz respiratoires se situent dans l'interface entre la surface intérieure de l'ouverture (40) et le contour extérieur (26) du raccord (21) et les éléments d'espacement (10) se trouvent entre la surface intérieure de l'ouverture (40) et le contour extérieur (26) du raccord (21), qui prédéfinissent la hauteur de dimensionnement des surfaces d'exhalation (7), **caractérisé en ce que** le raccord (21) fait partie intégrante de l'appui-front (2) et sa coque de masque (1) présente une ouverture ronde centrale (40), qui sert de logement à l'appui-front (2) et forme ainsi une interface (41) par rapport à l'appui-front (2).

2. Masque respiratoire selon l'une au moins des revendications précédentes, dont l'ouverture (40) est ronde et présente un diamètre intérieur (45) d'au moins 1 cm et/ou dont l'ouverture (40) présente une,longueur (47) d'au moins 4 mm.

3. Masque respiratoire selon l'une au moins des revendications précédentes, dont l'ouverture (40) de la coque de masque (1) est définie dans une zone par un tube (42) de diamètre constant et dont la zone tubulaire (42) est longue de plus de 4 mm.

4. Masque respiratoire selon l'une au moins des revendications précédentes, dont la zone tubulaire (42) se plie vers l'extérieur à un angle de 20 - 90° par rapport à la zone tubulaire et forme une zone conique (43) .

5. Masque respiratoire selon l'une au moins des revendications précédentes, qui présente plusieurs surfaces d'exhalation (7) sur la surface intérieure de l'ouverture (40), qui sont inter-rompues par des éléments d'espacement (10) tels que des nervures et dont les éléments d'espacement (10) s'étendent de l'intérieur vers l'extérieur le long de la surface intérieure de la coque de masque (1) et dirigent ainsi les gaz respiratoires du patient vers l'extérieur.

6. Masque respiratoire selon l'une au moins des revendications précédéntes, dont l'appui-front (2) est exécuté sous la forme d'une unité avec un logement articulé (27) destiné à une pièce de raccordement (4).

7. Masque respiratoire selon l'une au moins des revendications précédentes, dont la structure d'exhalation des gaz expirés est définie par les surfaces de l'ouverture (40) de la coque de masque (1) et de l'appui-front (2).

8. Masque respiratoire selon l'une au moins des revendications précédentes, dans lequel au moins deux canaux (8) sont disposés sur le pourtour de l'ouverture (40), qui sont réalisés sous la forme d'un évidement dans la zone tubulaire et/ou dans la zone conique et dans lequel les canaux (8) font office de logements pour l'accouplement mécanique de l'appui-front (2), qui peut se faire par exemple sous la forme d'une fermeture à baïonnette, sachant que les canaux (8) servent à une double fonction tant comme des logements pour l'accouplement mécanique de l'appui-front (2) que comme canaux d'échappement des gaz respiratoires expirés.

9. Masque respiratoire selon l'une au moins des revendications précédentes, dans lequel les canaux (8) font office de logements pour l'accouplement mécanique de l'appui-front (2), qui est réalisé sous la forme d'une fermeture à baïonnette et dans lequel une dent (9) est prévue respectivement pour la fermeture à baïonnette dans la zone arrière près de chaque canal (8), qui dépasse radialement vers l'intérieur du contour intérieur de l'ouverture (40) et qui oppose la résistance mécanique requise à la fermeture.

10. Masque respiratoire selon l'une au moins des revendications précédentes, dans lequel l'enclenchement mécanique s'effectue entre la coque de masque et l'appui-front dans le sens radial moyennant des éléments d'encliquetage disposés radialement par rapport au contour intérieur de l'ouverture (40).

11. Masque respiratoire selon l'une au moins des revendications précédentes, dans lequel plusieurs nervures d'espacement (10) sont disposées sur la surface intérieure de l'ouverture (40), qui permettent de relier la coque de masque (1) à l'appui-front (2) sans jeu ou sous une tension initiale.

12. Masque respiratoire selon l'une au moins des revendications précédentes, dans lequel les nervures d'espacement (10) sont de l'ordre de 100 - 500 *µ*m de haut et sont dimensionnées de sorte qu'elles forment une fente pour les gaz respiratoires ou dans lequel les nervures d'espacement (10) présentent des hauteurs différentes ou une augmentation graduelle de la hauteur ou dans lequel les nervures d'espacement (10) sont moins hautes vers l'intérieur dans la zone tubulaire (42), par exemple de l'ordre de 100 - 299 *µ*m, et plus hautes dans la zone conique (43) par exemple de l'ordre de 300 - 500 *µ*m.

13. Masque respiratoire selon l'une au moins des revendications précédentes, dans lequel le matériau est renforcé en fonction de la hauteur des nervures d'espacement (10) dans la zone annulaire et conique (42, 43) au niveau (11) des canaux d'échappement (8) et dans lequel les nervures d'espacement (10) et les zones renforcées (11) limitent latéralement les surfaces d'exhalation (7) .

14. Masque respiratoire selon l'une au moins des revendications précédentes, dans lequel des structures visibles et/ou palpables (13) sont constituées dans la coque de masque, qui s'écartent radialement des canaux d'échappement et indiquent ainsi le sens d'échappement des gaz respiratoires.
